(19) Europäisches Patentamt
European Patent Office
Office européen des brevets

(11) **EP 4 209 168 A1**

(12) **EUROPEAN PATENT APPLICATION**

(43) Date of publication:
**12.07.2023 Bulletin 2023/28**

(21) Application number: **22305011.3**

(22) Date of filing: **07.01.2022**

(51) International Patent Classification (IPC):
***A61B 3/028*** *(2006.01)*     ***A61B 3/032*** *(2006.01)*

(52) Cooperative Patent Classification (CPC):
**A61B 3/032**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Essilor International**
**94220 Charenton-Le-Pont (FR)**

(72) Inventors:
- **BOUTINON, Stéphane**
  **75013 PARIS (FR)**
- **DE ROSSI, Hélène**
  **94100 SAINT MAUR DES FOSSES (FR)**

(74) Representative: **Plasseraud IP**
**66, rue de la Chaussée d'Antin**
**75440 Paris Cedex 09 (FR)**

(54) **APPLICATION FOR MYOPIA MONITORING**

(57)     A method for monitoring an evolution of a visual acuity value of a user (1) of a digital device (2) configured for displaying optotypes at a chosen optotype size, to be guessed by said user (1), and computing a guessing rate corresponding to a percentage of optotypes correctly identified by the user (1), the method comprising:
a) determining a visual acuity reference value (VA_ref) based on at least a predefined first guessing rate threshold (THS) of the displayed optotypes at an optotype reference size (OS_ref),
b) testing the evolution of the visual acuity value by:
b1) displaying optotypes at a first optotype size corresponding to the optotype reference size (OS_ref),
- measuring the guessing rate of the displayed optotypes, and, if the measured guessing rate is below the first guessing rate threshold (THS) by more than a tolerance value (ε) :

b2.1) remeasuring the guessing rate of displayed optotypes at a second optotype size (OS2.1), said second optotype size (OS2.1) being superior to the first optotype size.

EP 4 209 168 A1

FIG. 6

**Description**

The present invention relates to the field of visual acuity assessment and more specifically to myopia monitoring using a mobile application.

In order to assess the visual acuity of an individual, an appointment with an eye specialist is often required. Complex apparatus are usually used and specific measures are performed by the eye specialist in order to detect a possible refractive shift and thus a change of visual acuity of the individual. Moreover, between two different appointments, which are often spaced by several months, there is no simple method for the individual in order to evaluate his or her visual acuity and its evolution. In particular, the evolution of the visual acuity of an individual enables to identify potential refractive errors (e.g., myopia, hyperopia, astigmatism) and/or to monitor the evolution of such refractive errors. In the case of myopia, which progression may be significant for children in particular, a visual acuity monitoring may enable to regularly check the visual acuity of the individual and to detect a degradation or a stabilization of myopia. Indeed, a degradation of the myopia of an individual is reflected in a degradation of his or her visual acuity. A visual acuity monitoring also enables, in the case of specific eye treatments aiming for example at curbing the eye degradation and/or slowing the evolution of refractive errors, to evaluate the efficiency of such eye treatments.

Existing solutions for determining the visual acuity of an individual do not enable to provide a simple, efficient and accurate method to be self-performed on a regular basis by the individual.

Therefore, there is an issue regarding the possibility for an individual to monitor the evolution of his or her visual acuity in order to monitor the evolution of potential refractive errors such as myopia, in an accessible, simple yet accurate way.

The present invention addresses such problems.

It is proposed, in a first aspect of the invention, a method for monitoring an evolution of a visual acuity value of a user of a digital device configured for:

-    displaying optotypes at a chosen optotype size, to be guessed by said user, and
-    measuring a guessing rate corresponding to a percentage of optotypes correctly identified by the user,
     the method comprising:

     a) determining a visual acuity reference value, said visual acuity reference value being determined based on at least a predefined guessing rate threshold of the displayed optotypes at an optotype reference size,

     b) testing the evolution of the visual acuity value by:

     -    displaying optotypes at a first optotype size corresponding to the optotype reference size,

     -    measuring the guessing rate of the displayed optotypes,

     -    and, if the measured guessing rate is below the guessing rate threshold by more than a tolerance value:
          b2.1) remeasuring the guessing rate of displayed optotypes at a second optotype size, said second optotype size being superior to the first optotype size.

As a consequence, an individual is able to measure an evolution of his or her visual acuity without requiring any complex apparatus or an appointment with an eye specialist. By using a basic digital device, the individual is able to perform a visual acuity test and iterating such visual acuity test on a daily, weekly or monthly basis in order to evaluate an evolution of his visual acuity throughout time. In particular, the myopia monitoring method provides a modulated iteration of the visual acuity tests which is based on challenging the last updated level of visual acuity of the individual. Thus, it is possible, at each new visual acuity test, to detect a potential degradation of the visual acuity of the individual between two successive visual tests. Such method is particularly relevant for myopic users such as myopic children since the myopia of children often progresses rapidly and requires a frequent monitoring. The myopia monitoring may thus be performed in a simple and regular way, for example at home with a personal or shared digital device such as a mobile phone, a tablet or a computer. Moreover, the use of a psychometric function which links the guessing rate of optotypes and the visual acuity provides an accessible and easily-understandable method to be performed by child users for example. Such method may thus be performed on a daily or weekly basis for example. Plus, the tested level of visual acuity and thus the modulation of the optotype size of the displayed optotypes being computed and updated by the digital device over time, the user is not required to set any initialization data or to provide any specific medical input such as his visual acuity evolution record for example.

Moreover, the method enables to obtain data related to an evolution of the visual acuity of the individual. By measuring such evolution via measured, remeasured guessing rates corresponding to specific optotype sizes (and thus, specific visual acuity values), the resulting data provided by the proposed method are easily interpretable, for example for the individual (e.g., a child) and/or for other persons (e.g., eye specialists, relatives of the individual such as parents, etc.).

By visual acuity, it is understood a measure of the spatial resolution that a human eye is able to perceive. That is, when two points are spaced by a sufficient distance for a given human eye, such human eye is able to distinguish two separated points. Yet, when such distance is too small, the human eye is not able to distinguish two different points. The quantification of such sufficient distance corresponds to the visual acuity. More generally, the visual acuity of a human eye may be defined based on the minimal size, or angular size, of an optotype that an individual is able to reliably identify. Such minimal angular size is referred to as the "Minimum Angle of Resolution" (or MAR) and is expressed in minutes of angle (or arcmin).

A visual acuity value, or level of visual acuity, may then be defined as a decimal value or a fraction corresponding to the inverse of the minimum angle of resolution. For example, if a MAR of an individual is 1 arcmin, the visual acuity of the individual is 1.0, that is 10/10. If a MAR of an individual is 4 arcmin, the visual acuity of the individual is ¼, that is 2,5/10.

An optotype, or eye chart, may be understood as a graphical symbol, drawing or letter which is displayed to the individual at a predefined optotype size. The optotype may be a Landolt-C or a letter on the Snellen eye chart for example. The optotype size, also referred to as an optotype detail size, may be understood as an angular size (expressed in arcmin) at which the individual perceives the displayed optotype. Such angular size is defined considering notably a distance of the individual from the symbol to be read. The optotype size may also be understood as an absolute size of the optotype on the displaying surface, expressed in millimeters. The angular size of an optotype may be deduced based on the absolute size of the optotype and the distance of the individual from the optotype to be read via the tangent formula.

As a consequence, considering a fixed physical distance between a position of the individual and the surface on which the optotype is displayed, the optotype size of a displayed optotype corresponds to a uniquely tested minimum angle of resolution (or MAR) and is thus associated to a unique tested level of visual acuity.

By displaying optotypes, it is understood that the digital device displays a series of several optotypes on a screen of the digital device. The digital device may be held by the user and positioned by the user so that the screen of the digital device may face a reflective surface such as a plane mirror. The user is thus able to see the optotypes displayed on the mirror as a virtual image. The screen may also be positioned to face the user, so that the displayed optotypes are directly seen by the user on the screen. The optotypes are displayed to the user as a series of optotypes of the same size, the optotypes of a same series of optotypes being displayed in a successive way. The series of optotypes may also be displayed on a same line by the digital device.

By a guessing rate, it is understood a percentage or a ratio of a number of optotypes that are correctly guessed or identified by the user on a total number of optotypes in a series of optotypes displayed to the user. One guessing rate is defined for one series of optotypes displayed at a given optotype size. That is, one guessing rate may be associated to one tested level of visual acuity. A maximal guessing rate is 100 % of optotypes being correctly identified by the user. A minimal guessing rate is the probability of blindly guessing the correct optotype : for example, if a displayed Landolt-C has eight random directions, the probability of randomly guessing the correct direction of the displayed Landolt-C is 1/8 or 12,5 %.

By a guessing rate threshold, it is understood a predefined and fixed value of the guessing rate and which defines a satisfying percentage of good answers. That is, when being displayed a series of optotypes at a given optotype size, if the user reaches a guessing rate equaling or close to the guessing rate threshold, the level of visual acuity corresponding to the given optotype size is considered to be the accurate level of visual acuity of the user at the time of the test. Indeed, when the guessing rate is far above the guessing rate threshold (for example, by more than a predefined tolerance value), the tested optotype size may be bigger than the actual minimum angle of resolution of the user (in other words, the user may be able to identify smaller optotypes with a still satisfying accuracy). When the guessing rate is far below the guessing rate threshold, it is considered that the optotype size is below the minimum angle of resolution of the user which leads to an inability of the user to accurately guess the displayed optotypes. In an embodiment, the guessing rate threshold is comprised between 50 percent and 70 percent.

By a tolerance value, it is understood a tolerated percentage of error of the guessing rate within which the guessing rate is considered to approximately correspond to the guessing rate threshold. In an embodiment, the tolerance value is comprised between 5 percent and 15 percent. For example, if the guessing rate threshold is 60 percent and the tolerance value is 10 percent, then all guessing rates comprised between 50 percent and 70 percent of correct guesses are considered to correspond to the guessing rate threshold. Such tolerance value enables to compensate the imprecision of the method, notably linked to the probabilistic aspect of the guessing rate measurement. The value of the tolerance value may be predefined depending notably on the number of optotypes displayed at a given optotype size. For example, the tolerance value may be preset at 10 percent for the guessing of ten displayed optotypes at a given optotype size and the tolerance value may be preset at 5 percent for the guessing of twenty displayed optotypes at a given optotype size.

By an optotype reference size, it is understood the optotype size for which the first guessing rate threshold is reached by the user when guessing the displayed optotypes at such optotype size. The optotype reference size thus corresponds to the minimum angle of resolution of the user. By a visual acuity reference value, it is understood a level of visual acuity which has been determined to be the accurate level of visual acuity of the user at the time of the test. Such visual acuity reference value may be defined as the inverse of the optotype reference size. The visual acuity reference value defines the visual acuity of the user at a specific time. The visual acuity of the user may change over time with respect to such visual acuity reference value or, contrarily, may be stabilized to such visual acuity reference value.

By measuring a guessing rate, it is understood that the digital device is able to collect and process the answers of the user in response to a displayed series of optotypes at a given optotype size so as to determine a guessing rate associated to a tested optotype size (that is, a tested level of visual acuity). For example, the user may vocally express his or her answer at each displayed optotype, such vocal answer being received by a microphone of the digital device for example. The user may also make a gesture in order to indicate his or her answer, such gesture being received by a front camera of the digital device. The displayed optotypes may also include a movable cursor that the user is able to control using physical switches on the digital device (such as the plus and minus buttons of a mobile device). In such case, the answer of the user is received by the camera which detects the position of the movable cursor with a predetermined latency.

The user is understood as the individual using the digital device in order to monitor his or her own visual acuity evolution. The user may perform the monitoring method with or without wearing an eye correction (such as corrective glasses or lenses) and for one eye (in the context of a monocular vision test) or for both eyes (in the context of a binocular vision test).

By a visual acuity test, it is understood the method performed at a given time consisting notably in displaying a series of optotypes at a fixed optotype size to the user by the digital device and in determining the guessing rate corresponding to such optotype size by the digital device based on the receiving of answers from the user in response to the displayed optotypes.

By remeasuring the guessing rate of displayed optotypes at a second optotype size, said second optotype size being superior to the first optotype size, it is understood that a new series of optotypes with a new optotype size (here, the second optotype size) is displayed to the user, so that a new guessing rate (here, the remeasured guessing rate) is determined based on the answers provided by the user in response to such new series of optotypes. The second optotype size is not determined randomly : such second optotype size is superior to the first optotype size, meaning that an inferior level of visual acuity is tested on the user compared to the immediately preceding visual acuity test performed on the user. Such method relies on the assessment that a decreasing ability to correctly identify optotypes reflects a decreasing level of visual acuity of the myopic user.

The new series of optotypes at the second optotype size may be displayed to the user after a predetermined time duration with respect to the immediately preceding displayed series of optotypes. Such time duration may be one or several days. By spacing distinct visual acuity tests in time, an accurate evolution of the visual acuity may be obtained. In the case of eye monitoring in order to observe the effects of a specific eye treatment, spacing distinct visual acuity in time enables to obtain the mid and long-term effects of the treatment on the vision of the individual.

It is proposed, in a second aspect of the invention, a digital device comprising a communication interface comprising at least a screen, a human/machine interface and a processing circuit configured to implement the method according to the first aspect of the invention.

Such digital device enables its user to perform a self-monitoring method in a simple and accessible way. Indeed, such digital device may be handled by the user. Such digital device may for example correspond to a tablet or a smartphone of the user. Advantageously, the proposed method may be performed using a digital device that is daily used by the user and that the user has easy access to.

It is proposed, in a third aspect of the invention, a server, connected to a communication network and comprising a storage memory storing a database for the implementation of the method according to an embodiment of the first aspect of the invention, said database being accessible by at least one terminal and providing at least an history of an evolution of a visual acuity value to a user of said terminal.

Such server may be a distant server such as a cloud platform. The server may receive data and signaling from any digital device connected to the communication network. As a consequence, the server may advantageously centralize information and data collected and measured by the digital device. The server may also be used as a relay in order to supply the data provided by the proposed method to other individuals at distance from the user of the digital device and who may have an interest and/or an ability to interpret the data collected by the digital device.

It is proposed, in a fourth aspect of the invention, a computer program product comprising program instruction code stored on a computer-readable medium for the execution of the method according to the first aspect of the invention.

According to an aspect of the invention, the method further comprises:

if the measured guessing rate is superior to the guessing rate threshold by more than the tolerance value:
b2.2) remeasuring the guessing rate of displayed optotypes at a third optotype size, said third optotype size being inferior to the first optotype size.

As a consequence, the method enables to provide a modulated monitoring, notably when it is detected that the farsightedness of the user has progressed over time. In such case, a third optotype size of optotypes is tested in the next visual acuity test performed on the user. Such third optotype size corresponds to a higher level of visual acuity compared to the level of visual acuity tested at the immediately preceding visual acuity test.

According to an aspect of the invention, the method further comprises:
if the gap between the measured guessing rate and the guessing rate threshold is inferior or equal to the tolerance value:
b2.3) remeasuring the guessing rate of displayed optotypes at a same optotype size as the first optotype size.

As a consequence, the method enables to test the stability of the myopia of the user by repeating the visual acuity test at the same tested level of visual acuity. Such new visual acuity test is performed after a predetermined time duration with respect to the precedent visual acuity test, such predetermined time duration being for example several days.

Indeed, when, at a given time, the measured guessing rate for displayed optotypes at the first optotype size results in a guessing rate corresponding approximately to the guessing rate threshold, it is considered that the tested level of visual acuity (corresponding to the first optotype size) is the accurate level of visual acuity of the user at the given time. In other words, it is considered that the guessing rate of the user when being displayed optotypes at the first optotype size at the current time does not differ much from the guessing rate of the user, would the user being displayed optotypes at the optotype reference value. A next visual acuity test is then performed later in order to test if such visual acuity reference value remains relevant.

In an embodiment, the guessing rate is remeasured after a predefined time. In an embodiment, such predefined time is superior or equal to seven days.

Advantageously, the monitoring method is performed by spacing different testing of the visual acuity values in time, so that an accurate evolution of the visual acuity of the user may be observed.

In an embodiment, the method further comprising:
c) determining the evolution of the visual acuity value of the user by comparing the visual acuity reference value with at least another visual acuity value, said another visual acuity value being determined by the digital device and related to the same optotype size as the first optotype size, the second optotype size, or the third optotype size, depending on the measured guessing rate.

In an embodiment, the method further comprises :
d) monitoring an evolution of data related to at least one refractive error of the user based on the evolution of the visual acuity value of the user.

Advantageously, different values of visual acuity tested during the proposed method may be compared in order to obtain an evolution, or a recorded history, of the visual acuity value of the user throughout time. In particular, the proposed method enables to obtain data relevantly collected according to the evolution of the visual acuity of the user, such data being interpretable in order to deduce negative and/or positive effects on the eyes of the user of various treatments, factors etc.

In an embodiment, steps b2.1), b2.2) and b2.3) are repeated through a plurality of iterations, said first optotype size being replaced at each new iteration either by the same optotype size as the first optotype size, the second optotype size or the third optotype size, depending on the guessing rate measured at the immediately preceding iteration.

Advantageously, the proposed method adapts the visual acuity tests to the performance of the user in reaction to the displayed optotypes. As a consequence, the method provides a modulable and adaptative process to observe the visual acuity evolution of the user so as to detect any refractive error, change of eye correction, defocus for example.

In an embodiment, said repetition ends when at least one or a combination of the following conditions is met:

- a preset maximum number of iterations is met, and

- the first optotype size is replaced by the same optotype size for a predefined number of successive iterations.

As a consequence, the duration of the monitoring phase of the myopia monitoring method may be flexible and may depend on the user criteria and goals. Such duration may be fixed by a maximum number of iterations of the visual acuity test, in the case of a myopia monitoring performed between two appointments with the eye specialist for example. The myopia monitoring is thus performed over a predetermined number of days, weeks or months so that results of myopia monitoring may be provided to the eye specialist. The myopia monitoring method may also aim at determining an accurate level of visual acuity of the user at different times and observe its progression, for example in the case of an eye treatment followed by the user and/or in order to observe the evolution of myopia of a child user for example. A level of visual acuity may also be identified as accurate when the visual acuity of the user is measured to be stabilized at such level of visual acuity for a predefined number of visual acuity tests performed over a regular time.

The monitoring method may also be performed until another appointment with an eye specialist occurs for the user.

According to an aspect of the invention, the visual acuity reference value is determined by:

- displaying a plurality of successive optotypes at a plurality of successive corresponding optotype sizes,

- measuring, for each corresponding optotype size of the displayed optotypes among the plurality of displayed optotypes, a corresponding guessing rate, at least one guessing rate being superior to the first guessing rate threshold and at least one other guessing rate being inferior to the first guessing rate threshold,

- identifying a displayed optotype for which the corresponding guessing rate corresponds to the guessing rate threshold, the corresponding optotype size corresponding to the optotype reference size and the visual acuity reference value being related to said corresponding optotype size.

As a consequence, the myopia monitoring process first consists in a preliminary phase, during which the visual acuity reference value is determined. The determination of such visual acuity reference value is performed through a plurality of visual acuity tests. Advantageously, such method enables to obtain an accurate value of visual acuity of the user, without requiring complex equipment or an appointment with an eye specialist.

In an embodiment, the displaying of the plurality of successive optotypes and the measurement of each corresponding guessing rate respect a predetermined periodicity, said periodicity being superior or equal to one day.

In an embodiment, any two successive corresponding optotype sizes have different values, a ratio between such values corresponding to a factor determined by a predetermined factor function. The factor function may also be a constant function equaling the factor.

Advantageously, the determination of the accurate value of visual acuity of the user consists in testing several distinct values of visual acuity in order to frame the actual accurate value of visual acuity.

In an embodiment, the visual acuity reference value is determined by performing an interpolation search on the plurality of successive optotypes displayed at the plurality of successive corresponding optotype sizes and on the corresponding guessing rates.

Indeed, the guessing rate threshold being predefined and known, it is possible to determine the optotype size for which the measured guessing rate corresponds the first guessing rate threshold, among the plurality of tested optotype sizes. Such determined optotype size corresponds to the optotype reference value and the corresponding level of visual acuity is defined as the visual acuity reference value.

In an embodiment, the digital device is positioned by the user so that the displayed optotypes are reflected on a mirror facing the user. The method then comprises, before measuring and/or remeasuring the guessing rate of displayed optotypes, :

collecting surrounding data related to current conditions of use of the digital device, such surrounding data comprising at least one or a combination of :

- a distance between a position of the digital device and a virtual image of the digital device created by the reflection of the digital device on the mirror,

- a level of ambient light, and

- a timestamp associated with the displayed optotypes,

Plus, the displayed optotypes depend on the collected surrounding data.

As a consequence, the monitoring method guarantees an accuracy of the results since the successive visual acuity tests are performed on the user in the same conditions and the results of such visual acuity tests are thus comparable.

By surrounding data, it is understood data received from a human/machine interface of the digital device. The digital device used for performing the monitoring method may be equipped with a camera in order to evaluate a distance between the digital device and a reflective surface on which the displayed optotypes are read by the user, such as a mirror. The digital device may also be equipped with an ambient light sensor so that the digital device may be able to examine the brightness of light in the area where the visual acuity test is performed. The user may also vocally express information about his or her positioning, the time of the day or any other information about the set-up for performing the visual acuity test, such information being collected and processed by speech recognition for example. The surrounding data may also include the monocular or binocular nature of the monitoring.

By the displayed optotypes depend on the collected surrounding data, it is understood that the displaying optotypes at the predetermined optotype size to the user (that is, before starting the visual acuity test) depend on a controlling, by the digital device, that the detected, received and/or processed surrounding data meets the set-up conditions for performing the visual acuity test. Such set-up conditions may be predefined as initialization data included in the application for myopia monitoring or may correspond to the surrounding data collected at the first visual acuity test performed on the user. If such set-up conditions are not met by the surrounding data, the digital device may display, via a communication interface of the digital device, a notification or a signal informing the user that the conditions for performing the visual acuity test are not met. For example, the digital device may display on the front screen of the digital device that the user is too far or too close with respect to the mirror compared to a set-up positioning, or that the lighting is insufficient compared to the set-up lightning. The digital device may also store in a memory unit, a periodicity to be respected between two successive visual acuity tests and may inform the user to iterate the visual acuity test later if it is determined that the periodicity is not respected compared to the time of the last visual acuity test. When the surrounding data is detected to meet the set-up conditions (with a predefined margin of error for example), the visual acuity test may start and the optotypes are displayed by the digital device.

In an embodiment, the visual acuity reference value and the optotype reference size are determined at a first time, the displayed optotypes at the first optotype size and the measured guessing rate are determined at a second time, the displayed optotypes at the second optotype size and the remeasured guessing rate are determined at a third time. At least one storage memory is then provided and:

- data related to the visual acuity reference value and/or to the optotype reference size, are stored in said storage memory in correspondence to data of said first time, and
- data related to the first optotype size and to the measured guessing rate, are stored in said storage memory in correspondence to data of said second time, and
- data related to the second optotype size and to the remeasured guessing rate, are stored in said storage memory in correspondence to data of said third time, and

said data is furthermore stored in correspondence to an identifier of the user.

As a consequence, the method enables to timestamp each visual acuity test with conditions of visual acuity testing and an identifier of the user, so that all data related to the method are recorded and arranged. Such data may thus be formatted in order to be transmitted to other devices for example for interpretation of further processing.

In an embodiment, said storage memory stores data of a database and is accessible through a communication network, by at least one terminal so as to provide at least a history of the evolution of said visual acuity value to a user of said terminal.

In an embodiment, output data of the digital device comprises, for a given user:

- a visual acuity reference value,

- chosen optotype sizes of displayed optotypes, and

- guessing rates of the displayed optotypes,

the digital device being connected to a communication network and configured to transmit said output data, through said communication network, to a distant server.

As a consequence, the results of the myopia monitoring and the successive visual acuity tests are registered to be transmitted, interpreted and accessed via a storage memory. Such storage memory may be supported by the server, on a digital platform via a cloud or by a digital device. Thus, for each user monitoring his or her visual acuity via the monitoring method, data related to the evolution of the visual acuity of the user may be consulted and interpreted by the ophthalmologist of the user, his or her parents, his optician or any relevant eye care professional (or ECP) for example. Such ECP may also rely on the data resulting from the proposed method for establishing an eye diagnosis of the user. The database of the storage memory may be updated by the data transmitted by a digital device in real time, after a visual acuity test ends or when the monitoring phase ends for example. The database may thus centralize data related to the myopia monitoring of a plurality of users and an eye care history of each user may be consulted by requests performed on the database using the identifier of the user.

Thus, the results of the monitoring method may be merged with information and knowledge of eye care specialists in order to obtain a complete and exhaustive eye care history (or record) of the user. The server may also process the data stored in the storage memory in order to obtain curbs of evolution of the myopia of users and establish statistical metrics about the efficiency of a specific eye treatment or about the progression of myopia by children for example.

By a user of a terminal, it is understood any individual or organism that has an interest in obtaining or consulting the results of the myopia monitoring of users via such terminal. Such user of a terminal may correspond to the user himself or herself (that is, the user performing the myopia monitoring) in order to consult his or her own results, his or her parents, an ophthalmologist or any eye care professional (ECP). The terminal may refer to a digital device of the user of the terminal such as a computer or a mobile device for example.

Other features, details and advantages will be shown in the following detailed description and on the figures, not in a limitative way, on which:

- [Fig. 1] represents a schematic usage of a digital device for testing a visual acuity value on the user.

- [Fig. 2] represents a flowchart showing steps of a visual acuity test on the user using a digital device.

- [Fig. 3] represents a flowchart showing steps for a preliminary phase of determining the visual acuity reference value.

- [Fig. 4] represents an evolution of the guessing rate of two individuals depending on the optotype size.

- [Fig. 5] represents a flowchart showing steps of a monitoring phase according to an embodiment of the invention.

- [Fig. 6] represents results of a myopia monitoring process according to an embodiment of the invention.

- [Fig. 7] represents a digital device according to an embodiment of the invention.

- [Fig. 8] represents a system for transmitting results of myopia monitoring according to an embodiment of the invention.

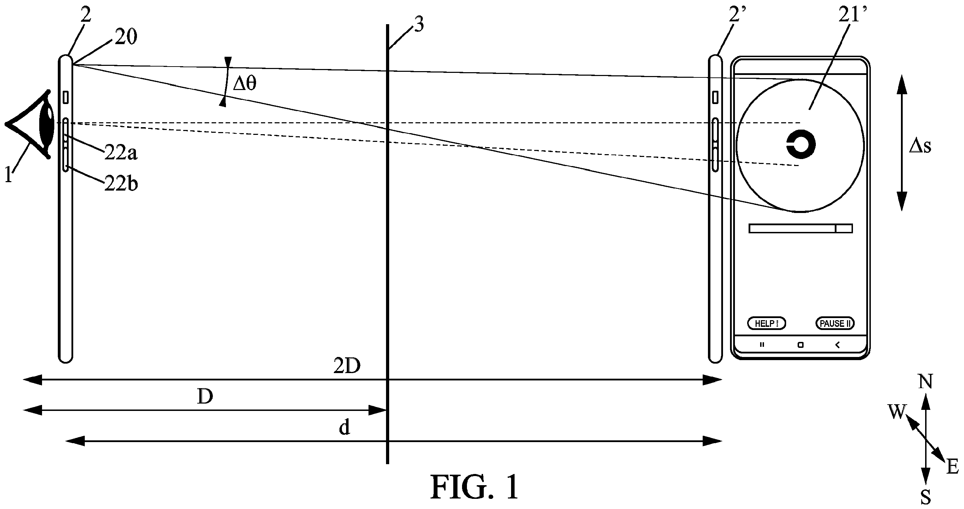

It is now referred to figure 1. Figure 1 represents a set-up for testing the visual acuity of a user 1 of the set-up in a punctual way, using a digital device 2 and a reflective surface such as a mirror 3, the mirror being plane.

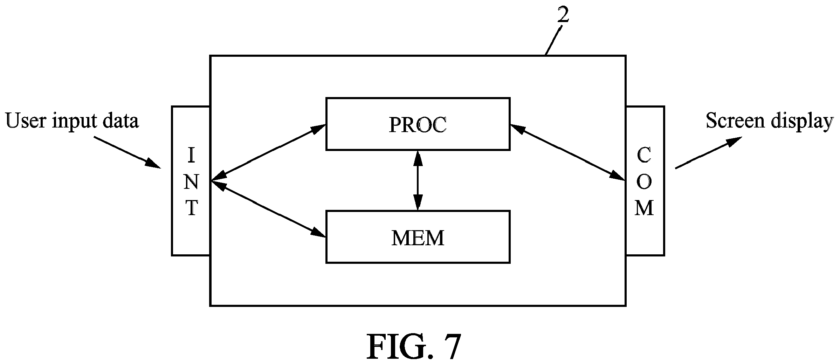

The digital device 2 refers to a portable computer device that can be used independently, and small enough to be easily carried by the user 1 and operated in the hand. Such digital device 2 may for example refer to a smartphone, a phablet, or a tablet. As illustrated on figure 7, the digital device 2 comprises at least a processing unit PROC, a memory unit MEM and an output communication unit COM, which comprises at least a screen. More generally, the digital device 2 comprises a processing circuit including the processing unit PROC. The processing circuit includes a computer program product which comprises program instruction code stored on a computer-readable medium of the product, so that the digital device 2 is able to execute steps for testing the visual acuity of a user 1 and for monitoring the evolution of such visual acuity, as detailed further in the description.

The output communication unit COM may also comprise a speaker. The digital device 2 also comprises a human/machine interface INT so that the digital device 2 is able to collect data received from the user 1 and from the environment surrounding the digital device 2. Such human/machine interface INT may include one or several of the following elements: a speech recognition module, a gesture and/or image recognition module (via a camera for example), a front camera 20, physical switches on the digital device 2 to be pressed by the user 1 (such as plus and minus volume switches for example), a tactile surface. The screen and the front camera 20 of the digital device 2 may be positioned on the same face of the digital device 2. The digital device 2 also comprises communication means so that the digital device 2 is able to emit and/or receive radiofrequency signals to and/or from devices and other equipments in the surrounding environment of the digital device 2. For example, the digital device 2 is able to communicate via Bluetooth, Wi-Fi and/or 3G/4G/5G protocols.

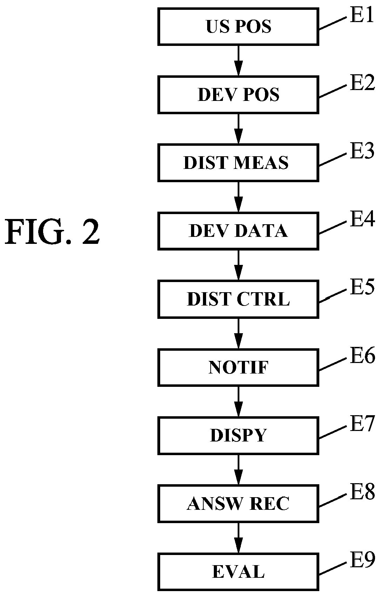

It is now referred to figure 2. Figure 2 illustrates successive steps for performing a visual acuity test on the user 1 of the digital device 2, using such digital device 2.

In order to test his or her visual acuity, the user 1 may first proceed to a user positioning step E1. During the user positioning step E1, the user 1 of the digital device 2 positions himself or herself in front of a mirror 3 at a predefined distance D from the mirror 3. As illustrated in figure 1, the user 1 is positioned at a predefined distance D facing the mirror 3 to see the virtual images 2', 21' created by the light reflected on the surface of the mirror 3. The mirror 3 is a plane mirror. When the user 1 is positioned at the predefined distance D, the distance between said user 1 and the virtual image the user 1 sees in the mirror 3 may be defined as equal to two time the predefined distance D. According to an embodiment, the predefined distance D is greater than or equal to 1.5 meters, preferably greater than or equal to 2.0 meters. Advantageously, the predefined distance D is greater than or equal to 2.5 meters to better relax accommodation. With such conditions, the visual acuity test evaluates the visual acuity of the user 1 for far vision. Alternatively, the predefined distance D may be smaller than or equal to 0.5 meter, preferably greater than or equal to 0.25 meter. With such conditions, the visual acuity test evaluates the visual acuity of the user 1 for near vision. In the context of the present description, measurements of visual acuity are performed in the context of myopia monitoring. The visual acuity of the user 1 is thus tested for far vision.

The user 1 may then proceed to a positioning step of the digital device 2 E2. During the positioning step of the digital device 2 E2, the user 1 positions the digital device 2 to have the screen of the device 2 facing the mirror 3. Preferably, the digital device 2 is positioned vertically to be substantially parallel to the plane of the mirror 3. In an embodiment, the digital device 2 may be positioned over one eye of the user 1, as represented on figure 1, and the visual acuity of the other eye (i.e., the eye uncovered by the digital device 2) is evaluated. Advantageously, such positioning of the digital device 2 facilitates the evaluation of the monocular visual acuity of the user 1.

The visual acuity test then comprises a distance measuring step E3. During such distance measuring step E3, the distance d between a front camera 20 of the digital device 2 and a virtual image 2' of the digital device 2 is measured. Such distance d may be measured by the digital device 2 itself.

The visual acuity test then comprises a digital device data processing step E4, during which data related to the digital device 2 is collected. Such data related to the digital device 2 may comprise data related to the physical size of the screen of the digital device 2 and camera data related to at least the angular resolution of the front camera 20's pixel. The physical size of the screen of the digital device 2 may be expressed as $w_s$ x $h_s$, with $w_s$ being the physical screen's width and $h_s$ the physical screen's height. Such physical size may be obtained based on the resolution of the screen $m_s$ x $n_s$ (with $m_s$ being the width of the screen in pixels and $n_s$ being the height on the screen in pixels) and the pixel density of the screen $D_{p,s}$ (expressed in dots per inches or dpi). The resolution and the pixel density of the screen can be easily obtained from the mobile device's operating system (OS) or from manufacturer data. Knowing the values ms, ns, and Dp,s, the values of ws and hs can be determined using the following equation:

[Math. 1]

$$w_s = \frac{m_s}{D_{p,s}}$$

$$h_s = \frac{n_s}{D_{p,s}}$$

By way of non-limiting example, the calculation of the screen's physical size of a mobile device 2 is exemplified for a specific smartphone with a screen resolution ms x ns equal to 1440 x 3040 and a pixel density Dp,s equal to 550 dpi. Using equation [Math. 1], we obtain a physical screen's width ws equal to 66.5 millimeters and a physical screen's height equal to 140.4 millimeters. Alternatively, the physical screen's size can be calculated from the diagonal physical size of the screen and the resolution.

The angular resolution of the front camera 20's pixel $p_{c,\theta}$ can be obtained from a front camera 20's pixel size $p_c$ (expressed in meters) and a focal length $f_c$. Similarly, the front camera 20's pixel size and the focal length can be obtained from the digital device's operating system (OS) or from manufacturer data. Knowing the values $p_c$ and $f_c$, the value $p_{c,\theta}$ can be determined using the following equation:

[Math. 2]

$$p_{c,\theta} = 2 arctan\left(\frac{p_c}{2f_c}\right) \approx \frac{p_c}{f_c}$$

By way of non-limiting example, the calculation of the angular resolution of the front camera 20's pixel of a mobile device 2 is exemplified for the specific smartphone with a front camera 20 pixel's size $p_c$ equal to 1.22 $\mu$m and a focal length equal to 3.34mm. Using equation [Math. 2], we obtain angular resolution of the front camera 20's pixel $p_{c,\theta}$ equal to 0.021 degrees.

For simplicity's sake, the camera's pixel physical size $p_c$ is considered to have the same value in horizontal and vertical directions. However, the reasoning could be easily extended if this condition is not true. In another embodiment, the value $f_c$ can be replaced by the real distance between a lens and a camera's sensor of the digital device 2 since the specified working distance of the front camera 20 is likely not set at infinity. Alternatively, the real distance between the lens and the camera's sensor can be calculated by assuming that the camera is set for a finite distance between 0.5 meter and 1.0 meter, preferably at 0.8 meter.

The distance d between the front camera 20 of the digital device 2 and the virtual image 2' created by the mirror 3 on which said digital device 2 is reflected may be measured based on the data related to the digital device 2. To that end, the processor PROC of the digital device 2 may collect data captured from the front camera 20. Typically, the front camera 20 of the digital device 2 captures images and/or videos of the mirror and of the virtual image 2' formed by the mirror 3 facing the front camera 20. The images and/or videos may further be processed to extract data relating to the size of the virtual image 2' of the screen of the digital device 2 reflected by the mirror 3. For example, the width of the screen of the virtual mobile device 2' reflected by the mirror 3 may be expressed as a number of pixels $m_{c,d}$ on a picture/video recorded by the front camera 20. Similarly, the height of the screen of the virtual mobile device 2' reflected by the mirror 3 may be expressed as a number of pixels $n_{c,d}$ on a picture/video recorded by the front camera 20. The distance d between the front camera 20 of the digital device 2 and the virtual image 2' created by the mirror 3 may then be determined using the following equations:

[Math. 3]

$$m_{c,d} \times p_{c,\theta} = arctan\left(\frac{w_s}{d}\right) \approx \frac{w_s}{d}$$

$$\rightarrow d \approx \frac{w_s}{m_{c,d} \times p_{c,\theta}}$$

Alternatively, the distance d may be measured by displaying on the screen of the digital device 2, different shapes of predefined known dimensions. Knowing the dimension of the displayed shapes, the distance d can be measured using equations of [Math. 2] and [Math. 3].

The visual acuity test may also comprise a distance controlling step E5. During the distance controlling step E5, the measured distance d between the front camera 20 of the digital device 2 and the virtual image 2' created by the mirror 3 is compared to the predefined distance D.

The visual acuity test may further comprise a notification step E6. During the notification step E6, a notification is sent to the user 1 by the digital device 2 based on the comparison of the measured distance d and the predefined distance D. For example, when the measured distance d between the front camera 20 of the digital device 2 and the virtual image 2' created by the mirror 3 is smaller than the predefined distance D, the notification may indicate that the user 1 is too close to the mirror 3. For example, when the measured distance d between the front camera 20 of the digital device 2 and the virtual image 2' created by the mirror 3 is greater than the predefined distance D, the notification may indicate that the user is too far to the mirror 3. Alternatively, a maximal deviation ΔD from the value of the predefined distance D may be defined. When the measured distance d is smaller than (2D-ΔD), the notification may indicate that the user 1 is too close to the mirror 3. Similarly, when the measured distance d is greater than (2D+ΔD), the notification may indicate that the user 1 is too far from the mirror 3. The maximal deviation ΔD may correspond to a variation of 20%, preferably 10%, more preferably 5% of the predefined distance D.

The visual acuity test further comprises a displaying step E7. Preferably, the displaying step E7 is performed after the distance controlling step E5 and the notification step E6. During the displaying step E7, optotypes are displayed on the screen of the digital device 2. In the sense of the disclosure, optotypes refer to standardized symbols commonly used to test the vision of a person.

As illustrated on figure 1, the optotypes displayed on the screen of the digital device 2 are reflected on the mirror 3 which generates virtual images 21' of the optotypes. Preferably, a symmetry is applied to the displayed optotype so that the orientations of the optotype as displayed on the screen of the digital device 2 and as seen by the user 1 are similar. Advantageously, the user 1 will perceive the virtual images 21' of the displayed optotypes with the correct orientation. The displayed optotypes may be Landolt-C or Snellen E or any other standardized optotypes, letters, or drawings. The optotypes may be displayed one by one (that is, successively) on the screen of the digital device 2 during the displaying step E7. Alternatively, optotypes may be displayed line by line with multiple optotypes on each line. The size and/or orientation and/or contrast of each optotype may vary during the displaying step. Alternatively, multiple lines of optotypes may be displayed at the same time, with optotypes of different size and/or orientation and/or contrast on each line, for example ETDRS charts like or 2-lines Essilor VR-800 procedures. Alternatively, the displayed optotypes may correspond to words or short sentences.

During the displaying step E7, the size of the displayed optotypes (referred to as the "optotype size") may be adapted based on the measured distance d. Alternatively, the size of the displayed optotypes may be determined based on the predefined distance D from the mirror 3. Similarly, the angular size (or detail size) of the displayed optotypes on the screen of the digital device 2 may be adapted based on the measured distance d.

By way of non-limiting example and as represented on figure 1, the optotype considered for the following calculation is a Landolt ring with an aperture so that the virtual image 2' of the displayed Landolt ring at a distance d of the digital device 2 has an angular size of Δθ. The physical size Δs of the aperture displayed on the screen is given by the following equation:

[Math. 4]

$$\Delta s = d \times tan(\Delta \theta)$$

For example, for a measured distance of 4.0m, the aperture of the displayed Landolt ring $\Delta \theta$ for a visual acuity of 10/10 should be equal to 1'. Using equation [Math. 4], the physical size $\Delta s$ of the aperture displayed on the screen is approximatively equal to 1.16mm. Referring to the specific smartphone taken as an example, a physical size $\Delta s$ of the aperture displayed on the screen equal to 1.16mm corresponds to a number of pixel ($\Delta s/ps$) approximately equal to 25 pixels on the screen.

The displayed optotypes 21' may comprise a single Landolt-C as represented on figure 1, with a Landolt-C gap directed in a random direction among several predetermined directions. Such predetermined directions may refer to the four cardinal directions (north N, south S, east E and west W) and/or the ordinal directions (northeast, southeast, northwest and southwest) for example.

According to an embodiment of the disclosure, the distance d between the front camera 20 and the mirror 3 is frequently measured during the displaying step. For example, the distance d may be measured before displaying a different optotype or series of optotypes. Preferably, the distance d between the front camera 20 and the mirror 3 is measured in real time. Advantageously, it allows updating regularly, for example in real time, the size and/or the angular size of the displayed optotypes on the screen of the digital device 2, thereby providing a more accurate testing of the visual acuity of the user 1.

The visual acuity test further comprises an evaluation step E9. During the evaluation step E9, the accuracy of the tested visual acuity on the user 1 is evaluated.

The method for evaluating the accuracy of the tested visual acuity on the user 1 may further comprise, prior to the evaluation step E9, an answer receiving step E8. During the answer receiving step E8, answers, or indications (more generally, input data) are received from the user 1 in response to each displayed optotype. Such input data may be received by the digital device 2 via the human/machine interface INT. The input data of the user 1 may be received using speech recognition and/or gesture recognition and/or mobile device movement. When the optotypes displayed on the screen of the digital device 2 are words or short sentences, the user 1 may identify the letter or read the sentences. These indications received from the user 1 may be obtained by the processing circuit of the digital device 2 using speech recognition, for example through a microphone of the digital device 2. When the optotypes displayed on the screen of the digital device 2 are Landolt-C with an aperture having different directions, as illustrated on figure 1, the user 1 may indicate the direction of the aperture he perceived. The indication relating to the direction of the aperture perceived by the user 1 may be obtained by speech recognition using a microphone of the digital device 2, mobile device movement using an IMU of the digital device 2 or the front camera of the digital device 2, and head movement and/or arm movement using the front camera of the digital device 2. The digital device 2 may further comprise switches such as physical plus ("+") and minus ("-") volume buttons 22a and 22b. Preferably, the plus volume button 22a and the minus volume button 22b are disposed on the side of the digital device 2. The plus and minus volume buttons 22a and 22b may be used by the user 1 to provide an indication during the answer receiving step. Such use of the physical switches of the digital device 2 is particularly advantageous in the case of displayed optotypes being Landolt-C type with an aperture having different directions. Such displayed optotypes may for example comprise a movable cursor. The movable cursor may be for example, a circular art that move along the different possible directions of the aperture of the Landolt-C. In such case, the user 1 may utilize the physical plus and minus buttons 22a and 22b of the digital device 2 to have the direction of the movable cursor aligned with the perceived aperture of the Landolt-C. The chosen position of the cursor may then be validated after a short waiting period, for example comprised between 0.5 second and 2.0 second, preferably after a waiting period of 1.0 second.

The accuracy of the tested visual acuity on the user 1 may be evaluated during the evaluation step E9 based on the optotype size of the optotypes displayed during the displaying step E7 and the received indications from the user 1 during the answer receiving step E8.

Indeed, during the displaying step E7, a first series of various randomized optotypes, for example corresponding to a same first level of visual acuity, is presented to the user 1. Such displaying of the first series of optotypes may consist in successively displaying a predefined number of optotypes within the first series of optotypes at a first optotype size corresponding to the first level of visual acuity. Such optotype size (and/or such first level of visual acuity being recorded in the memory unit MEM of the digital device 2). At each displayed optotype, the digital device 2 may receive input data from the user 1 through the human/machine interface INT indicating the optotype guessed by the user 1. For example, referring to figure 1, the correct answer (or indication) is a gap of the displayed Landolt-C oriented in the west W direction. Such input data is collected at the answer receiving step E8 for each displayed optotype of the first series of optotypes displayed during the displaying step E7. During the evaluation step E9, the indications received from the user 1 are evaluated and a score corresponding to the accuracy of the indications may be attributed to the user 1. Such score, also referred to as a "guessing rate", is, in particular, associated with the first optotype size of the displayed optotypes, and thus with the first level of visual acuity.

In the context of the present description, the visual acuity of a user 1 may quantify the ability of the user 1 to distinguish the smallest detail possible with a satisfying accuracy, a satisfying accuracy being defined as an ability to guess details (typically optotypes 21') displayed at a considered size (typically, an optotype size) with a percentage of good answers, also referred to as a "score", or a "guessing gate", being equal to a guessing rate threshold (THS). There is then a direct and existing correspondence between the visual acuity and the optotype size of the displayed optotypes, such correspondence notably depending on the distance at which such optotypes are displayed to the user 1. The distance d being regularly checked to be fixed, a test at a given optotype size or a test at a given level of visual acuity may thus be understood in an interchangeable way since an optotype size is associated to a unique level of visual acuity. The optotype size (or equivalently, the level of visual acuity) of the displayed optotypes being controlled by the digital device 2, the digital device 2 then measures the guessing rate of the user 2 in response to optotypes displayed at such optotype size.

The guessing rate of a user 1 when being displayed optotypes 21' at an optotype size may be defined by a ratio (or equivalently, a percentage) of a number of optotypes that may be correctly guessed by the user 1 over a total number of displayed optotypes. For example, if the guessing rate of a user 1 is equal to 1 (or 100 percent), it is considered that the user 1 is undoubtedly able to identify the optotypes displayed at a tested optotype size and the visual acuity corresponding to such tested optotype size majorizes the actual visual acuity of the user 1 (indeed, the user 1 may be able to identify optotypes at a smaller size than the tested optotype size). The guessing rate of a user 1 is considered to be minimal when it is impossible for the user 1 to guess the displayed optotypes 21' other than by a random guess. The minimal guessing rate is thus the probability of randomly guessing the correct optotype: for a Landolt-C with eight different positions, the minimal guessing rate is thus 1/8 or 12,5 percent.

The guessing rate threshold THS may be comprised between 50 percent and 70 percent, preferably approximately equal to 60 percent. In an embodiment, the visual acuity may also be defined as an ability to guess optotypes 21' displayed at a considered optotype size with a guessing rate approximately corresponding to the guessing rate threshold THS by a tolerance value $\varepsilon$. Such tolerance value $\varepsilon$ may be comprised between 5 percent and 15 percent. As a consequence, when the guessing rate of displayed optotypes of the user 1 is approximately equal to the guessing rate threshold THS (or different than the guessing rate threshold THS with a gap inferior to the tolerance value $\varepsilon$), the optotype size of the displayed optotypes during the visual acuity test may be considered as the smallest size of detail accurately perceivable by the user 1. The level of visual acuity associated to such optotype size is then determined to be the visual acuity of the user 1 at the time of the visual acuity test.

In another embodiment, the set-up for testing the visual acuity of the user 1 may consist in a digital device 2 corresponding to a computer. Such computer may include a screen facing the user 1 and which directly displays optotypes to the user 1. The user 1 then expresses his or her answers, by voicing or typing, in response to the displayed optotypes, such answers being recorded by the computer. The process may also take place in the context of a virtual appointment with an eye specialist.

In another embodiment, the visual acuity of the user 1 may be evaluated using another method, Preferably, the visual acuity of the user 1 is evaluated using a self-measurement method.

The visual acuity test as illustrated on figures 1 and 2 enables to test a specific visual acuity value and more particularly the accuracy of such visual acuity on the user 1, using a self-measurement method with a digital device 2. With a visual acuity test, a chosen (or tested) visual acuity value (and thus, a chosen optotype size) is tested on the user 1 by the displaying of a series of optotypes at such chosen optotype size. Such tested visual acuity value may be the accurate value of visual acuity of the user 1 at the time the visual acuity test is performed. That is notably reflected in a guessing rate approximately corresponding to the guessing rate threshold THS. Yet, if the guessing rate computed during the evaluation step E9 is significantly different from the guessing rate threshold THS (for example, a gap between the computed guessing rate and the guessing rate threshold THS exceeds the tolerance value $\varepsilon$), the tested visual acuity is considered inaccurate.

In order to monitor the evolution in time of a refractive error such as myopia, the proposed method consists in monitoring the evolution of the visual acuity value of the user 1, assuming that an evolution of the refractive error of the user 1 is reflected in an evolution of the visual acuity value of the user 1. The proposed method notably relies on performing several visual acuity tests in order to evaluate chosen visual acuity values on the user 1. In order to test a chosen visual acuity value on the user 1, the visual acuity test described in figures 1 and 2 may be applied. Alternatively, other existing methods for estimating a value related to a visual acuity of the user 1 may be applied. The Stanford Acuity Test (or StACT) or the Freiburg Visual Acuity Test (FrACT) are example of such existing methods.

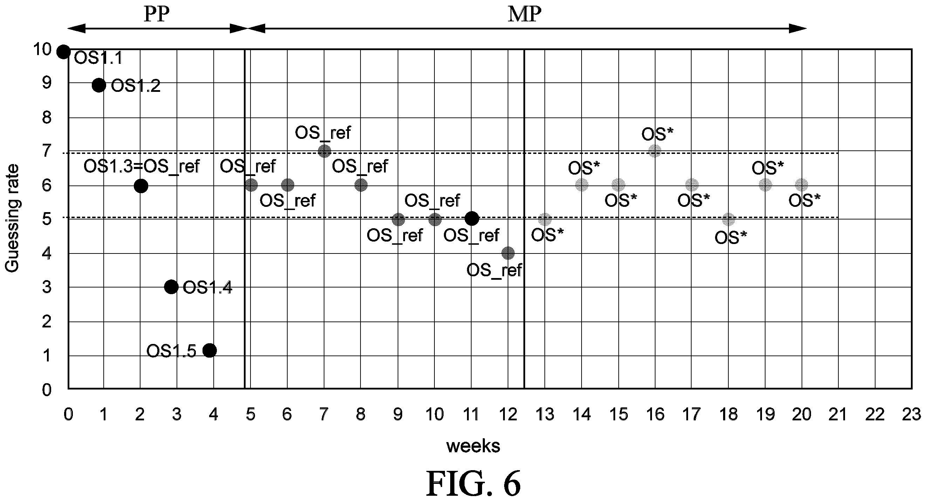

The proposed method to evaluate the evolution of the visual acuity of the user 1 consists in two distinct phases: a preliminary phase PP and a monitoring phase MP. A detailed application of the proposed method is shown on figure 6.

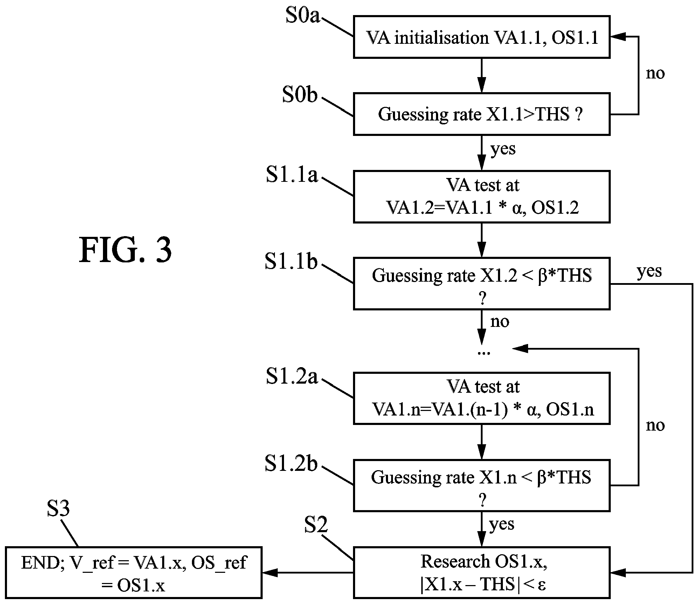

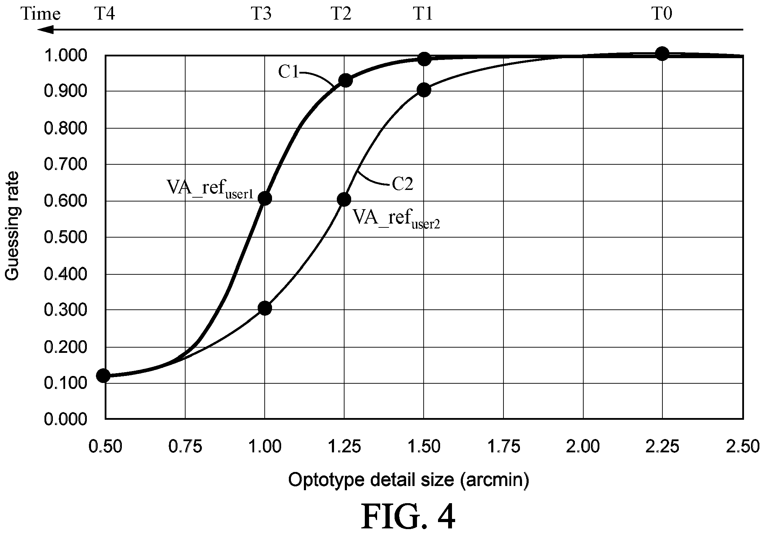

The preliminary phase PP consists in determining, at a given time, an accurate value of the visual acuity of the user 1, such accurate value of the visual acuity being referred to as the "visual acuity reference value" VA_ref. Such preliminary phase PP is detailed by figures 3 and 4.

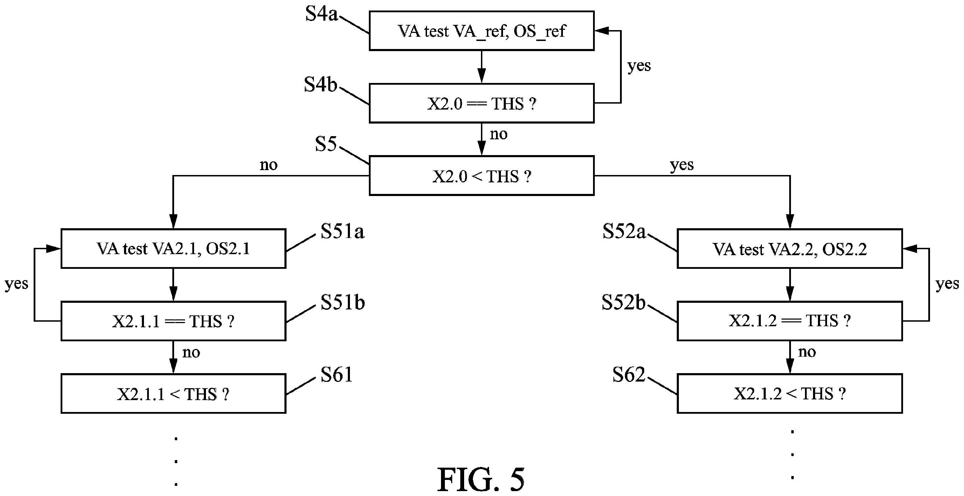

The monitoring phase MP then consists in monitoring, over time, an evolution of the visual acuity value of the user 1, with respect to the visual acuity reference value VA_ref. Such monitoring phase MP is detailed by figure 5.

In particular, such monitoring phase provides data enabling to monitor an evolution of refractive errors of the user 1 such as myopia. Indeed, if, throughout time, the value determined to be the accurate visual acuity of the user 1 decreases compared to the visual acuity reference value VA_ref, it means that the myopia of the user 1 has progressed over time (resulting in a degraded nearsightedness). If, throughout time, the value determined to be the accurate visual acuity of the user 1 is approximately close to the visual acuity reference value VA_ref (e.g., with a gap inferior to the tolerance value $\varepsilon$), it means that the myopia of the user has stabilized over time. In less common cases (for example, in the case of a particular myopia treatment assessment), if, throughout time, the value determined to be the accurate visual acuity of the user 1 increases compared to the visual acuity reference value VA_ref, it means that the myopia of the user 1 has decreased over time (resulting in an ameliorated nearsightedness). Moreover, based on the number of iterations of the visual acuity tests performed as well as the potential variations of the visual acuity values throughout time, data resulting from the monitoring phase MP may be interpreted to evaluate the progress of various eye diseases and/or the effects of various eye treatments.

It is now referred to figure 3. Figure 3 is a flowchart representing steps for determining the visual acuity reference value VA_ref of a user 1 during a preliminary phase PP of the myopia monitoring process, based on measures of the guessing rates for different visual acuity tests at different levels of visual acuity. In the context of the following figures, it is considered a guessing rate threshold THS predefined at 60 percent and a tolerance value $\varepsilon$ predefined at 10 percent.

At a step S0a, an initial value (or level) of visual acuity VA1.1 is tested for a user 1. Such value of visual acuity VA1.1 is for example chosen to be inferior to 1.0 (i.e., inferior to 10/10). Such initial level of visual acuity VA1.1 corresponds to a series of optotypes 21' displayed at an initial optotype size OS 1.1 by the digital device 2. For example, ten random optotypes at the same optotype size OS 1.1 are successively displayed on the screen of the digital device 2, such optotypes being for instance Landolt-C optotypes with a gap oriented in random directions among eight directions (cardinal and nominal directions). In other embodiment, the displayed series of optotypes may contain more or less than ten optotypes. At each display, the answer of the user 1 is received via the human interface machine INT of the digital device 2. After the ten optotypes at the optotype size OS 1.1 being displayed, a guessing rate X1.1 is determined in correspondence with the initial optotype size OS 1.1 (and thus with the initially tested level of visual acuity VA1.1). At a step S0b, such guessing rate X1.1 is verified to be superior to the guessing rate threshold THS, otherwise a lower initial level of visual acuity VA1.1 is tested (with a bigger optotype size OS1.1 compared to the tested optotype size OS1.1). In an embodiment, the guessing rate X1.1 is verified to be superior to the guessing rate threshold THS by more than a tolerance value $\varepsilon$. In other words, the guessing rate X1.1 is verified to exceed 70 percent. Indeed, the aim is to begin testing the visual acuity of the first user 1 with a low initial level of visual acuity with respect to the accurate visual acuity of the user 1 (also referred to as the visual acuity reference value VA_ref, which is unknown yet). The initial optotype size OS1.1 and the corresponding guessing rate X1.1 are then both stored in correspondence to the time of the initial visual acuity test in the memory unit MEM of the digital device 2.

At a step S1.1a, a new level of visual acuity VA1.2 superior to the initial visual acuity VA1.1 is tested for the user 1. Such step S1.1a may occur several days or a week after steps S0a and S0b. Such new level of visual acuity VA1.2 is tested via a series of optotypes displayed at an optotype size OS 1.2 smaller than the initial optotype size OS 1.1 by the digital device 2. The level of visual acuity VA1.2 (or equivalently, the optotype size OS1.2) may be determined based on the initial level of visual acuity VA1.1 (or equivalently, the initial optotype size OS 1.1) and a predetermined factor function f, so that VA1.2 = f(VA1.1). In an embodiment, such factor function may be a constant function equaling $\alpha$ (with $\alpha > 1$). Equivalently, a predefined factor function g may be applied to the optotype size OS 1.1 so as to obtain a new optotype size g(OS1.1) = OS 1.2 to be tested, such new optotype size OS 1.2 being smaller than the optotype size OS 1.1.

Such visual acuity test with optotypes displayed at the optotype size OS 1.2 results in a new measured guessing rate X1.2. In particular, such new measured guessing rate X1.2 is inferior or equal to the preceding guessing rate X1.1. Indeed, it is assessed that, for a myopic user 1, decreasing optotype sizes (and equivalently, increasing levels of visual acuity) lead to decreasing guessing rates. The guessing rate X1.2 is determined in correspondence with the optotype size OS1.2 (and thus with the tested level of visual acuity VA1.2). At a step S1.1b, such guessing rate X1.2 is compared to $\beta \ast$ THS, where $\beta$ is a predefined coefficient inferior or equal to 1. If such guessing rate X1.2 is superior to $\beta \ast$ THS, the testing and comparing steps S1.1a, S1.1b are repeated with a new level of visual acuity superior to the level of visual acuity VA1.2 (or equivalently, with an optotype size smaller than the optotype size OS 1.2) and determined based on the predetermined factor function f (or equivalently, based on the predefined factor function g) as described. The optotype size OS 1.2 and the corresponding guessing rate X1.2 are both stored in the memory unit MEM of the digital device 2, as well as the successive values of the optotype size and the respective corresponding guessing rates.

When, at an iteration n (n being an integer superior or equal to 2), the series of optotypes displayed at an optotype size OS1.n results in a guessing rate X1.n at a step S1.2a and such determined guessing rate X1.n is inferior to β * THS at a step S1.2b, the iteration stops. Indeed, with the condition X1.n < β * THS being met at step S1.2b, it is guaranteed that the process of figure 2 has been repeated enough times (here, n times) in order to bound the guessing rate threshold THS between two guessing rates in correspondence with two distinct optotype sizes OS1.1 and OS1.n. That is, the guessing rate threshold THS is comprised in an interval between the initial guessing rate X1.1 and the guessing rate X1.n, both guessing rates X1.1, X1.n being in correspondence with an optotype size OS 1.1, OS1.n. Based on the assumption that decreasing optotype sizes of displayed optotypes leads to decreasing guessing rates in the case of a myopic user 1, the process enables to bound the optotype size at which the guessing rate corresponds to the guessing rate threshold THS between the optotype sizes OS1.1 and OS1.n. In another embodiment, the stopping condition at step S1.2b also comprises one or a combination of the following elements:

- a predetermined fixed number of iterations of visual acuity testing,

- the gap between the guessing rate and the guessing rate threshold THS exceeds the tolerance value ε,

- the guessing rate is below the guessing rate threshold THS (β = 1 in this case).

Steps S0a, S0b, S1.1a, S1.1b, S1.2a, S1.2b thus consist in initializing the process of determining the visual acuity reference value VA_ref with an optotype size OS 1.1 for which the guessing rate exceeds the guessing rate threshold THS and then repeating the steps S1.1a and S1.1b with decreasing values of the optotype size until a guessing rate is sufficiently low with respect to the guessing rate threshold THS (that is, by a factor β for example).

At a step S2, it is thus possible to determine an estimation of the optotype size at which the guessing rate exactly corresponds to the guessing rate threshold THS, such optotype size being referred to as the optotype reference size OS_ref. In particular, such optotype reference size OS_ref is comprised between optotype sizes OS 1.1 and OS1.n, since the guessing rate threshold THS is comprised between guessing rates X1.1 and X1.n. If the guessing rate threshold THS has been reached during the process (that is, if during the iterative process, an optotype size OS1.x of displayed optotypes has led to a guessing rate corresponding to the guessing rate threshold THS), the optotype reference size OS_ref may be directly identified as the optotype size OS1.x at which the displayed optotypes have been guessed with a guessing rate equaling the guessing rate threshold THS. A search algorithm such as an interpolation search or a binary search may also be applied between the optotype sizes OS 1.1 and OS1.n in order to determine the optotype size OS1.x for which the guessing rate exactly corresponds to the guessing rate threshold THS. In another embodiment, at step S2, the optotype reference size OS_ref is chosen among as an optotype size OS1.x of displayed optotypes for which a gap between the corresponding guessing rate X1.x and the guessing rate threshold THS is below the tolerance value ε. In such case, if a plurality of values of optotype size fit to be the optotype reference size OS_ref, a median or mean value of such plurality may be determined to be the optotype reference size OS_ref.

At a step S3, such optotype size OS1.x is then defined as the optotype reference size OS_ref and the level of visual acuity corresponding to such optotype size OS1.x is defined as the visual acuity reference value VA_ref of the user 1.

The process of figure 3 is applied as an example and results in the curbs of figure 4.

Figure 4 represents the evolution of the guessing rate of two different users 1 depending on the optotype size of the optotypes 21' displayed to the respective users 1. The first curb C1 corresponds to the evolution of the guessing rate of a first user 1 to displayed optotypes 21' and the second curb C2 corresponds to the evolution of the guessing rate of a second user 2 to the same displayed optotypes 21'. The display and measures of the guessing rates for both users 1 may for example be performed according to the set-up of figure 1 and to the visual acuity test method of figure 2 and in similar conditions: both users 1 have the same positioning with respect to the mirror 3, predefined distances D and d are the same, the time (or at least the frequency) and the lightning conditions for the measures are similar. The iterative process of figure 3 is considered to stop for a user 1 if the guessing rate of the user 1 is strictly inferior to β*THS, with β = 1/3 and THS = 0.6 (or 60 percent). In other words, the iterative process is stopped when the guessing rate X1.x is strictly inferior to 0,2 (or 20 percent).

At an initial time T0, a same series of optotypes is displayed to both users 1 at a same initial optotype size OS1.1 = 2,25 arcmin. Based on the data input of users 1 received from the digital device 2, it is determined that both the first and the second users 1 have a same guessing rate X1.1 = 1 (or 100 percent). Such guessing rate X1.1 being superior to the guessing rate threshold THS (considered at 0,6 or 60 percent), it is guaranteed that the searched optotype reference sizes OS_ref$_{user\ 1}$, OS_ref$_{user\ 2}$ of both users 1 are inferior to OS 1.1 = 2,25 arcmin. At a time T1 later than time T0 (several days or a week later than time T0 for example), a new series of optotypes is displayed to both users 1 at a same optotype size OS 1.2 = 1,50 arcmin, corresponding to a higher level of visual acuity compared to the initial level of visual acuity tested at time T0. Such optotype size OS1.2 may be computed by the processing unit of the digital device 2 based on the initial optotype size OS 1.1 and a predetermined factor function g (defined here as a constant function g). For the optotype size OS1.2 = 1,50 arcmin, the first user 1 and the second user 1 have different guessing rates. As shown by the first curb C1, the first user 1 has a guessing rate X1.2$_{user\ 1}$ almost equal to 1 (or 100 percent) while the second user 1 has a guessing rate $X1.2_{user\ 2}$ of 0.9 (or 90 percent). Both guessing rates $X1.2_{user\ 1}$, $X1.2_{user\ 2}$ being superior to $\beta*THS = 20$ percent, the process is iterated at a time T2. At time T2 later than time T1, a new series of optotypes is displayed to both users 1 at a same optotype size OS 1.3 = g(OS1.2) = 1.25 arcmin. For the optotype size OS 1.3 = 1.25 arcmin, the first user 1 and the second user 1 have different guessing rates. As shown by the first curb C1, the first user 1 has a guessing rate $X1.3_{user}\ 1$ approximately equal to 0,92 (or 92 percent) while the second user 1 has a guessing rate $X1.3_{user\ 2}$ of 0.6 (or 60 percent). Both guessing rates $X1.3_{user\ 1}$, $X1.3_{user\ 2}$ being superior to $\beta*THS = 20$ percent, the process is iterated at a time T3. At time T3 later than time T2, a new series of optotypes is displayed to both users 1 at a same optotype size OS1.4 = g(OS1.3) = 1.00 arcmin. For the optotype size OS 1.4 = 1.00 arcmin, the first user 1 and the second user 1 have different guessing rates. As shown by the first curb C1, the first user 1 has a guessing rate $X1.4_{user\ 1}$ approximately equal to 0.60 (or 60 percent) while the second user 1 has a guessing rate $X1.4_{user\ 2}$ of 0.30 (or 30 percent). Both guessing rates $X1.4_{user\ 1}$, $X1.4_{user\ 2}$ being superior to $\beta*THS = 20$ percent, the process is iterated at a time T4. At time T4 later than time T3, a new series of optotypes is displayed to both users 1 at a same optotype size OS 1.5 = g(OS 1.4) = 0.50 arcmin. For the optotype size OS1.5 = 0.50 arcmin, the first user 1 and the second user 1 have a same guessing rate X1.5 equal to 0.125 (or 12.5 percent). It may be noted that such guessing rate X1.5 = 0.125 arcmin corresponds to the probability of randomly guessing the correct optotypes. Therefore, it means that for the optotype size OS1.5 = 0.50 arcmin, both users 1 are *a priori* unable to actually identify the displayed optotypes and have a complete random guess of the displayed optotypes. The guessing rate X1.5 being inferior to $\beta*THS = 20$ percent, the iteration is stopped.

In the example of figure 4, it is noted that the guessing rate threshold THS = 60 percent is reached for the first user 1 at the optotype size OS1.4 = 1.00 and for the second user 1 at the optotype size OS 1.3 = 1.25. No search algorithm is thus required and it may be determined that the optotype reference size of the first user 1 is $OS\_ref_{user\ 1}$ = 1.00 and that the optotype reference size of the second user 2 is $OS\_ref_{user\ 2}$ = 1.25. In particular, it is noted that, for the reference time T4, the visual acuity reference value $VA\_ref_{user\ 1}$ of the first user 1 is superior to the visual acuity reference value $VA\_ref_{user\ 2}$ of the second user 1 since it has been determined in the process that in similar testing conditions and considering the same required accuracy (that is the common guessing rate threshold THS), the first user 1 is able to identify smaller details (here, details of 1 minute of arc or 1 arcmin) compared to the second user 1 (here, details of 1.25 minutes of arc).

Values of predetermined function f, predefined function g, predefined coefficient $\beta$ are preset values. The values provided in the description of figures 3 and 4 are examples provided in a non-limitative way. In particular, by adapting such values, the search for the visual acuity reference value VA_ref may be more or less precise since more or less measures will be performed.

In another embodiment, the determination of the visual acuity reference value VA_ref may also result from an appointment with an eye specialist such as an ophthalmologist, wherein a level of visual acuity of the user 1 is identified at the time of the appointment, such level of visual acuity being considered as the visual acuity reference value VA_ref of the user 1 for debuting the monitoring phase MP.

It is now referred to figure 5. Figure 5 is a flowchart representing steps of a process for monitoring the visual acuity value (reflecting an evolution of a refractive error such as myopia) of the user 1 throughout time during a monitoring phase MP. Such monitoring phase MP consists in evaluating a possible evolution of the visual acuity of the user 1 with respect to a visual acuity reference value VA_ref of the user 1. Such visual acuity reference value VA_ref is determined at a time prior to the start of the monitoring phase MP during the preliminary phase PP as described on figure 3 for example. For example, the monitoring phase MP debuts one week after determining the visual acuity reference value VA_ref during the preliminary phase PP. The testing conditions of the visual acuity tests during the preliminary phase PP are applied to the visual acuity tests during the monitoring phase MP: the surrounding environment of the user 1 and the conditions of use of the digital device 2 in order to perform visual acuity tests are considered to remain the same. The guessing rate threshold THS is considered to be set at 60 percent and the tolerance value at 10 percent. In particular, a guessing rate value comprised between 50 percent and 70 percent of correct answers is considered to match the guessing rate threshold THS. Unless specified otherwise, "a guessing rate corresponding to the guessing rate threshold THS" or "a guessing rate approximately equal to the guessing rate threshold THS" means that the guessing rate is comprised between 50 percent and 70 percent (i.e., that the gap between the guessing rate and the guessing rate threshold THS does not exceed the tolerance value $\varepsilon$).

In particular, two successive visual acuity tests performed during the preliminary phase PP and/or the monitoring phase MP are spaced in time, so that an effective evolution of the visual acuity value of the user 1 may be observed. Preferably, successive visual acuity tests respect a time periodicity of one day, several days or weeks.

At a step S4a, the visual acuity of the user 1 is tested at the visual acuity reference value VA_ref: the digital device 2 is configured in the monitoring phase MP to display a series of (for example ten) optotypes at the optotype reference size OS_ref and a corresponding guessing rate X2.0 of the user 1 is measured in response to such series of displayed optotypes. In particular, step S4a is spaced in time with respect to the preliminary phase PP, so that the guessing rate X2.0 may potentially evolve with respect to guessing rate X1.x. At a step S4b, it is tested if such guessing rate X2.0 remains approximately equal to the guessing rate threshold THS. In other words, it is tested at step S4b if, when being displayed optotypes at the optotype reference size OS_ref, the user 1 still obtains a guessing rate corresponding to the guessing rate threshold THS, thus meaning that the visual acuity reference value VA_ref remains the accurate visual acuity value of the user 1 at the time step S4b is performed. Thus, at step S4b, it is tested if a gap between the guessing rate X2.0 and the guessing rate threshold THS remains below the tolerance value ε.

If the guessing rate X2.0 corresponds to the guessing rate threshold THS at step S4b, it means that the visual acuity of the user 1 at such step of the monitoring phase MP remains the same as his or her accurate visual acuity during the preliminary phase PP (in other words, the visual acuity reference value VA_ref *a priori* remains the accurate visual acuity of the user 1). In such case, after a fixed time duration lasting several days or weeks for example, step S4a is repeated and the visual acuity reference value VA_ref is tested again in order to confirm the stability of the vision of the user 1 or, in contrary, to detect a possible shift of vision. If, at step S4b, the guessing rate X2.0 is not equal to the guessing rate threshold THS (in other words, the gap between the guessing rate X2.0 and the guessing rate threshold THS exceeds the tolerance value ε), it means that the visual acuity of the user 1 has evolved compared to the preliminary phase PP and step S5 then occurs.

At step S5, it is determined whether the visual acuity (and probably the refractive error) of the user 1 has decreased or increased by comparing the guessing rate X2.0 with the guessing rate threshold THS, providing the tolerance value ε. If the guessing rate X2.0 is inferior to the guessing rate threshold THS (by more than the tolerance value ε), it may be understood that the myopia of the user 1 has increased at this time, resulting in a degraded visual acuity compared to the visual acuity reference value VA_ref of the user 1. In such case, after a fixed time duration lasting several days or weeks for example, a new step S51a of the monitoring phase MP is performed by taking such observed loss of visual acuity of the user 1 into account: the visual acuity of the user 1 is tested at a new level of visual acuity VA2.1, such new level of visual acuity VA2.1 being inferior to the visual acuity reference value VA_ref. In other words, a series of optotypes at a new optotype size OS2.1 is displayed by the digital device 2 to the user 1, such new optotype size OS2.1 being superior to the optotype reference size OS_ref. The determination of the optotype size OS2.1 is based on the immediate precedingly-tested optotype size (here, the optotype reference size OS_ref) and a predefined monitoring function G, so that OS2.1 = G(OS_ref). Such predefined monitoring function G may be a constant function consisting in multiplying optotype size values by a coefficient Ω, with Ω being strictly superior to 1. A new guessing rate X2.1.1 is then measured at step S51b in correspondence with the optotype size OS2.1 of the optotypes displayed at step S51a. The monitoring then consists in comparing such guessing rate X21.1 with the guessing rate threshold THS at steps S51b and S61, in a similar way to step S5, in order to determine the level of visual acuity to be tested in the next step (after the fixed time duration has passed and a new visual acuity test occurs for example). If, at step S51b, the guessing rate X2.1.1 corresponds to the guessing rate threshold THS, it means that the optotype size OS2.1 is considered to be the optotype size reflecting the actual level of visual acuity of the user 1 at the tested time. The next steps of the monitoring phase MP will consist in testing such optotype size OS2.1 in order to determine whether the visual acuity of the user has stabilized at the visual acuity VA2.1 or has evolved. If, at step S51b, the guessing rate X2.1.1 does not correspond to the guessing rate threshold THS, it means that the tested visual acuity VA2.1 does not correspond to the accurate visual acuity of the user 1 at the tested time : such actual visual acuity is still inferior to the visual acuity VA2.1 if, at step S61, the guessing rate X2.1.1 is inferior to the guessing rate threshold THS by more than the tolerance value ε and such actual visual acuity is superior to the visual acuity VA2.1 if, at step S61, the guessing rate X2.1.1 is superior to the guessing rate threshold THS by more than the tolerance value ε. The search of the actual visual acuity of the user 1 is pursued by repeating the precedent steps and replacing the guessing rate X2.0 with the new guessing rate X2.1.1 and the optotype reference size OS_ref with the new optotype size OS2.1.

At step S5, if the guessing rate X2.0 is superior to the guessing rate threshold THS (by more than the tolerance value ε), it may be understood that the myopia of the user 1 has decreased at the tested time of step S4a, resulting in an improved visual acuity compared to the visual acuity reference value VA_ref of the user 1. Such improvement of the visual acuity of the user 1 may for example occur in the case of specific myopia treatments undergone by the user 1 in order to cure myopia. In such case, after a fixed time duration lasting several days or weeks for example, a new step S51a of the monitoring phase MP is performed by taking the observed increase of visual acuity of the user 1 into account: the visual acuity of the user 1 is tested at a new level of visual acuity VA2.2, such new level of visual acuity VA2.2 being superior to the visual acuity reference value VA_ref. In other words, a series of optotypes at a new optotype size OS2.2 is displayed by the digital device 2 to the user 2, such new optotype size OS2.2 being inferior to the optotype reference size OS_ref. The determination of the optotype size OS2.2 is based on the immediate precedingly-tested optotype size (here, the optotype reference size OS_ref) and a predetermined monitoring function G', so that OS2.1 = G'(OS_ref). Such predetermined factor function G' may be a predetermined constant function consisting in multiplying optotype size values by a coefficient Ω', with Ω' being strictly inferior to 1. In other words, since the visual acuity is determined to have evolved in a positive way, such positive evolution is quantified by increasing the tested visual acuity value (that is, by decreasing the tested optotype size). In a similar way to steps S51b and S61, a new guessing rate X2.1.2 in correspondence with the optotype size OS2.2 is measured and steps S4b and S5 of the test are repeated by replacing X2.0 with X2.1.2 and VA_ref (and OS_ref) with VA2.2 (and OS2.2).

Predefined monitoring function G and predetermined monitoring function G' are respectively preset functions in order to change the tested values of optotype sizes in respective cases of degraded and upgraded visual acuity values of the user 1. At each iteration of the method of figure 5 during the monitoring phase MP, both functions G and ' may be successively used in order to determine the next optotype size to be tested. Depending on such functions G and G', the decreasing or increasing rate of tested optotype sizes may be adapted during the monitoring phase MP. Equivalently, monitoring functions may be applied to values of visual acuity instead of values of optotype sizes in order to test different values of visual acuity during the monitoring phase MP.

Such steps of the monitoring phase MP may be repeated over time in order to measure the evolution of myopia of a user 1. Such monitoring phase MP may end based on one or a combination of the following conditions :

- a maximum number of iterations of the visual acuity test is reached. This condition may be relevant when the monitoring is performed during a predetermined time between two appointments of the user 1 with an eye specialist for example. Based on a visual acuity reference value VA_ref determined at a first appointment, the user 1 may be able to obtain data related to the evolution of his or her myopia on a regular basis until the next appointment with the eye specialist.

- the guessing rate threshold THS is reached for a predefined number of consecutive iterations of the test. This condition sets a criterium for defining a stability of the visual acuity of the user 1. For example, at step S4b, if the guessing rate equals the guessing rate threshold THS for a number of successive displays of optotypes (for example, three, meaning that step S4a is repeated three consecutive times) at the optotype reference size OS_ref, it is considered that the visual acuity of the user 1 accurately and sustainably corresponds to the visual acuity reference value VA_ref and that the visual acuity of the user 1 has stabilized at the level of visual acuity VA_ref. The monitoring phase MP may thus be adapted, as it considered that the tested visual acuity of the user 1 is *a priori* accurate on the long term. Such adaptation may for example consist in increasing a time spacing between two successive visual acuity tests.

It is now referred to figure 6. Figure 6 represents an evolution of the measured guessing rate in response to series of optotypes displayed over time in the context of myopia monitoring of a user 1. Figure 6 details the data obtained by the digital device 2 when performing the proposed method, including the preliminary phase PP and the monitoring phase MP. Series of optotypes of different optotype sizes are displayed to the user 1 during a succession of visual acuity tests, during which a guessing rate of the user 1 is measured for each series of displayed optotypes. An accurate visual acuity of the user 1 is considered to be found when the user 1 is able to correctly identify displayed optotypes with a guessing rate corresponding to the guessing rate threshold THS preset at 0.60 (or 60 percent) providing the tolerance value $\varepsilon$ preset at 10 percent. In other words, a guessing rate comprised between the two dotted lines represented on figure 6 (corresponding to guessing rates comprised between 50 percent and 70 percent) is considered to correspond to the guessing rate threshold THS and the tested visual acuity (equivalently, the tested optotype size) is considered to be the accurate visual acuity (equivalently, the accurate optotype size) for the user 1.

Any two successive visual acuity tests are considered on figure 6 to be spaced by one week and are performed in similar testing conditions : the positioning of the user with respect to the mirror 3, the lightning conditions, the hour of the day, the tested eye in the case of a monocular vision testing for example, remain the same at each visual acuity test. In other embodiments, each visual acuity test may be spaced by more or less than one week. Visual acuity tests performed during the preliminary phase PP and the monitoring phase MP may be spaced by different times.

The process of myopia monitoring as represented on figure 6 consists in a preliminary phase PP between week 0 and week 4 and in a monitoring phase MP between week 5 and week 20.

During the preliminary phase PP, a visual acuity test is performed each week in order to determine the visual acuity reference value VA_ref of the user 1. The steps of such preliminary phase PP are detailed in figures 3 and 4 and the guessing rates of figure 6 actually correspond to results of the second user 1 (curb C2 of figure 4). After week 4, the preliminary phase PP ends and a search algorithm is performed on the five measures of guessing rates in order to determine the visual acuity reference value VA_ref of the user 1.

As detailed in the description of figures 3 and 4, the second tested visual acuity value is considered to be the accurate visual acuity value of the user 1 during the preliminary phase PP. In another embodiment, the visual acuity reference value VA_ref is determined by interpolation search on the measurements performed (notably in the case where guessing rates corresponding to the guessing rate threshold THS are not actually measured during the preliminary phase PP and have to be estimated).

The monitoring phase MP then begins at week 5 and consists in performing a succession of visual acuity tests in order to evaluate a possible evolution of the visual acuity of the user 1 with respect to the visual acuity reference value VA_ref of the user 1. The stopping condition of the monitoring phase MP is considered , in the example of figure 6, to be met if each measured guessing rate of the user 1 corresponds to the guessing rate threshold THS for eight consecutive weeks (which reflects a two-month stability of the visual acuity of the user 1). At week 5 (that is, one week after determining the visual acuity reference value VA_ref of the user 1), a visual acuity test is performed with a series of optotypes displayed to the user 1 at the optotype reference size OS_ref. In the example illustrated by figure 6, such visual acuity test results in a guessing rate corresponding to the exact value of the guessing rate threshold THS. The visual acuity test is thus repeated one week later, at week 6, with the same level of visual acuity (here VA_ref) being tested. As shown on figure 5, from week 5 to week 11, the resulting guessing rates of the consecutive visual acuity tests are exactly corresponding to the guessing rate threshold THS. Yet, the visual acuity test at week 12 at the visual acuity reference value VA_ref results in a guessing rate of 0.40 (or 40 percent), which is below the guessing rate threshold THS by more than the tolerance value $\varepsilon$. It is thus understood that after seven weeks of vision stability at the visual acuity reference value VA_ref (from weeks 5 to 11), a degradation of the visual acuity of the user 1 has been observed at week 12.

At week 13, a visual acuity test is thus performed at a different level of visual acuity VA* (or equivalently, a different value of optotype size OS*), such level of visual acuity being inferior to the visual acuity reference value VA_ref of the user 1. Such level of visual acuity VA* may be obtained by applying a monitoring function on the visual acuity reference value VA_ref or on the optotype reference value OS_ref (in such case, the predefined monitoring function G may be applied so as to test optotype size value OS*) in order to take the observed degradation of visual acuity at week 12 into account. Such monitoring function may be a constant function equaling a number strictly inferior to 1, so that the level of visual acuity VA* tested at week 13 is inferior to the visual acuity reference value VA_ref. The visual acuity test at week 13 thus consists in displaying optotypes at an optotype size OS* bigger than the optotype reference size OS_ref. The resulting guessing rate at week 13 is then observed to correspond to the guessing rate threshold THS. The monitoring phase MP is thus pursued with next visual acuity tests at the level of visual acuity VA* as long as the resulting guessing rate corresponds to the guessing rate threshold THS. At week 20, the guessing rate is measured to correspond to the guessing rate threshold THS for the eighth consecutive week (from week 13 to week 20). The monitoring process may thus conclude that a long-term accurate visual acuity value of the user is VA*. The process may also be pursued after week 20 by spacing visual acuity tests by more than one week for example. Such process may be pursued until a next appointment with an eye specialist for example.

An accurate measure of the visual acuity of the user 1 at week 20 is thus VA*. The myopia monitoring process thus enabled to evaluate that from week 0 to week 20, the user 1 encountered a loss of visual acuity, such loss being quantified (using the monitoring function used to obtain VA*, such loss being quantified by the ratio VA*/VA_ref) and delimited in time (it occurred between weeks 11 and 12). In another embodiment, the visual acuity tests may be spaced by more or less than one week, depending on the required time precision or on the age of the user 1 for example (a child usually has a more significant and rapid evolution of the vision compared to an adult and requires a more frequent myopia monitoring for example).

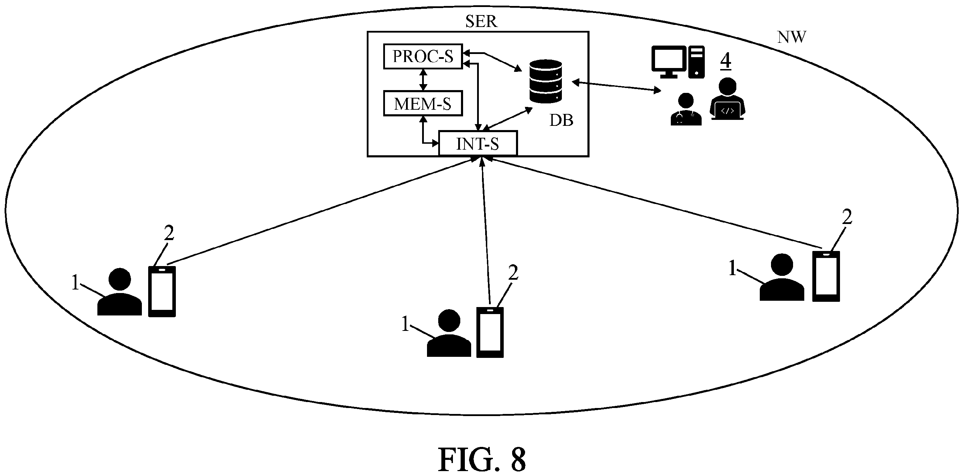

It is now referred to figure 8. Figure 8 represents a system for storing, processing and accessing data resulting from the proposed method. A digital device 2 as represented on figures 1, 7 and 8 may record data related to an evolution of the visual acuity value of one or several users 1 through a succession of visual acuity tests. Each acuity test is characterized by data related to, at least:

- a temporal information about the test, such as a time, a timestamp, an iteration, a day, a week of the test; and

- a tested level of visual acuity and/or a tested optotype size (or optotype detail size); and

- a resulting guessing rate of the user 1, which is determined based on input data received from the user 1.

Such data may be stored, for example temporarily, in a memory unit MEM of the digital device 2 at each iteration of the visual acuity test and associated with an identifier of the user 1. Such identifier is uniquely associated to the user 1 and may for example be a medical identifier (such as a social security number in the French health care system for instance) or a user account number of the myopia monitoring application. Such identifier may also relate to the digital device 2, for example in the case where each digital device 2 is used to monitor the visual acuity situation of a single user 1. Several digital devices 2 as represented on figure 8 may compute the monitoring process and store data related to the succession of acuity tests performed on each user 1 in their respective memory units MEM.

Moreover, each digital device 2 is connected to a communication network NW (including a wireless communication network for example) so that the digital devices 2 may send signals and data to a distant server SER of the network NW via a communication interface INT-S of the server SER. The server SER may be a cloud server for example. In particular, such server SER may include a storage memory which centralizes the data received from the digital devices 2 in a database DB of the storage memory. Such database DB may thus store data related to the visual acuity tests, the number of visual acuity tests, the visual acuity reference value VA_ref in correspondence with an identifier of each user 1. Such correspondence may for example be performed by a processing circuit (PROC-S, MEM-S) of the server SER. The database DB may also store a medical record of the user 1 regarding potential eye treatments or appointments for example. Such database DB may be updated in real time by the processing circuit (PROC-S, MEM-S) of the server SER, based on data received from the digital devices 2 after each visual acuity test and may be accessible to other individuals 4 via other terminals which are connected to the server SER via the communication network NW. As a consequence, the results of the monitoring process of users 1 may be communicated in real time to medical platforms, eye specialists or surroundings of the user 1 (such as the parents or the ophthalmologist of the user 1 for example) 4. In particular, the results of a myopia monitoring performed between two appointments with an eye specialist may be communicated in real time to the eye specialist (in the case of a surveillance of an eye treatment for example) or accessed by the eye specialist during the next appointment. Such results may then enable a simple and immediate medical interpretation or diagnosis of the refractive error (e.g., the myopia) of each user 1.

In a particular embodiment, the data transmitted by the digital devices 2 to the server SER are processed by the processing circuit (PROC-S, MEM-S) of the server SER before being stored in the database DB of the storage memory. For example, the server SER may stamp the date and time of the visual acuity test based on real-time data received from the digital device 2 or may record an iteration number of the visual acuity tests. The digital devices 2 may also directly send, for each visual acuity test, the tested optotype size and/or the answers of the user 1 in a raw form, to the server SER, which processes such data in order to deduce the tested level of visual acuity and/or the resulting guessing rate of the user 1. In another embodiment, the storage memory may also be directly included in a digital device 2, for example as part of the memory unit MEM of the digital device 2.

Reference key

1 : user of digital device
2 : digital device
20 : front camera of digital device
22a : physical switch, plus button of digital device
22b : physical switch, minus button of digital device
2' : virtual image of the digital device by reflection on the mirror
21' : virtual image of the displayed optotype by reflection on the mirror
3 : mirror
D : predefined distance between the user and the mirror
d : distance between the front camera of the digital device and the virtual image
$\Delta D$ : maximal deviation from the value of the predefined distance
THS : guessing rate threshold
$\varepsilon$ : tolerance value
VA_ref : visual acuity reference value
OS_ref : optotype reference size
OS2.1 : second optotype size
OS2.2 : third optotype size
PP : preliminary phase
MP : monitoring phase
X1.1, X1.x, X1.n: values of measured guessing rate during preliminary phase
VA1.1, VA1.2, VA1.n : values of tested visual acuity during preliminary phase
f : predetermined factor function for visual acuity
g : predefined factor function for optotype size
$\beta$ : predefined coefficient inferior to 1
X2.1.1, X2.1.2 : values of measured guessing rates during monitoring phase
OS2.1, OS2.2, OS* : values of tested optotype size during monitoring phase
VA2.1, VA2.2, VA* : values of tested visual acuity during monitoring phase
G : predefined monitoring function for optotype size
$\Omega$ : predefined constant value superior to 1
G' : predetermined monitoring function for optotype size
$\Omega'$ : predetermined constant value inferior to 1
4 : user of terminal
INT : human/machine interface of the digital device
PROC : processing unit of the digital device
MEM : memory unit of the digital device

COM : output communication unit of the digital device
DB : database included in a storage memory of the server
SER : server
(PROC-S, MEM-S) : processing circuit of the server
INT-S : communication interface of the server
NW : communication network

**Claims**

1. A method for monitoring an evolution of a visual acuity value of a user (1) of a digital device (2) configured for:

   - displaying optotypes at a chosen optotype size, to be guessed by said user (1), and
   - measuring a guessing rate corresponding to a percentage of optotypes correctly identified by the user (1),

   the method comprising:

   a) determining a visual acuity reference value (VA_ref), said visual acuity reference value (VA_ref) being determined based on at least a predefined guessing rate threshold (THS) of the displayed optotypes at an optotype reference size (OS_ref),
   b) testing the evolution of the visual acuity value by:
   b1) displaying optotypes at a first optotype size corresponding to the optotype reference size (OS_ref),

   - measuring the guessing rate of the displayed optotypes,
   - and, if the measured guessing rate is below the guessing rate threshold (THS) by more than a tolerance value ($\varepsilon$):
   b2.1) remeasuring the guessing rate of displayed optotypes at a second optotype size (OS2.1), said second optotype size (OS2.1) being superior to the first optotype size.

2. The method according to claim 1 further comprises:
   if the measured guessing rate is superior to the guessing rate threshold (THS) by more than the tolerance value ($\varepsilon$):
   b2.2) remeasuring the guessing rate of displayed optotypes at a third optotype size (OS2.2), said third optotype size (OS2.2) being inferior to the first optotype size.

3. The method according to claim 1 further comprises:
   if the gap between the measured guessing rate and the guessing rate threshold (THS) is inferior or equal to the tolerance value ($\varepsilon$):
   b2.3) remeasuring the guessing rate of displayed optotypes at a same optotype size as the first optotype size.

4. The method according to any one of the precedent claims, wherein the guessing rate is remeasured after a predefined time.

5. The method according to claim 4, wherein said predefined time is superior or equal to seven days.

6. The method according to claim 3 taken in combination with claim 2, further comprising:
   c) determining the evolution of the visual acuity value of the user (1) by comparing the visual acuity reference value (VA_ref) with at least another visual acuity value, said another visual acuity value being determined by the digital device (2) and related to the same optotype size as the first optotype size, the second optotype size (OS2.1), or the third optotype size (OS2.2), depending on the measured guessing rate.

7. The method according to claim 6 further comprising:
   d) monitoring an evolution of data related to at least one refractive error of the user (1) based on the evolution of the visual acuity value of the user (1).

8. The method according to any one of the preceding claims, wherein the guessing rate threshold (THS) is comprised between 50 percent and 70 percent.

9. The method according to any one of the preceding claims, wherein the tolerance value ($\varepsilon$) is comprised between 5 percent and 15 percent.

**10.** The method according to claim 3 taken in combination with claim 2, wherein steps b2.1), b2.2) and b2.3) are repeated through a plurality of iterations, said first optotype size being replaced at each new iteration either by the same optotype size as the first optotype size, the second optotype size (OS2.1) or the third optotype size (OS2.2), depending on the guessing rate measured at the immediately preceding iteration,

**11.** The method according to claim 10, wherein said repetition ends when at least one or a combination of the following conditions is met:

- a preset maximum number of iterations is met, and
- the first optotype size is replaced by the same optotype size for a predefined number of successive iterations.

**12.** The method according to any one of the preceding claims, wherein the visual acuity reference value (VA_ref) is determined by:

- displaying a plurality of successive optotypes at a plurality of successive corresponding optotype sizes,
- measuring, for each corresponding optotype size of the displayed optotypes among the plurality of displayed optotypes, a corresponding guessing rate, at least one guessing rate (X1.1) being superior to the guessing rate threshold (THS) and at least one other guessing rate (X1.n) being inferior to the guessing rate threshold (THS),
- identifying a displayed optotype for which the corresponding guessing rate (X1.x) corresponds to the guessing rate threshold (THS, the corresponding optotype size corresponding to the optotype reference size (OS_ref) and the visual acuity reference value (VA_ref) being related to said corresponding optotype size.

**13.** The method according to claim 12, wherein the displaying of the plurality of successive optotypes and the measurement of each corresponding guessing rate respect a predetermined periodicity, said periodicity being superior or equal to one day.

**14.** The method according to any one of claims 12 and 13, wherein any two successive corresponding optotype sizes have different values, a ratio between such values corresponding to a factor determined by a predetermined factor function (G).

**15.** The method according to any one of claims 12 to 14, wherein the visual acuity reference value (VA_ref) is determined by performing an interpolation search on the plurality of successive optotypes displayed at the plurality of successive corresponding optotype sizes and on the corresponding guessing rates.

**16.** The method according to any one of the precedent claims, the digital device (2) being positioned by the user (1) so that the displayed optotypes are reflected on a mirror (3) facing the user (1),
said method comprising, before measuring and/or remeasuring the guessing rate of displayed optotypes, :

collecting surrounding data related to current conditions of use of the digital device (2), such surrounding data comprising at least one or a combination of :

- a distance (d) between a position of the digital device (2) and a virtual image (2') of the digital device (2) created by the reflection of the digital device (2) on the mirror (3),
- a level of ambient light, and
- a timestamp associated with the displayed optotypes,

and wherein the displayed optotypes depend on the collected surrounding data.

**17.** The method according to any one of the precedent claims,

the visual acuity reference value (VA_ref) and the optotype reference size (OS_ref) being determined at a first time,
the displayed optotypes at the first optotype size and the measured guessing rate being determined at a second time,
the displayed optotypes at the second optotype size (OS2.1) and the remeasured guessing rate being determined at a third time,

wherein at least one storage memory is provided and:

- data related to the visual acuity reference value (VA_ref) and/or to the optotype reference size (OS_ref), are stored in said storage memory in correspondence to data of said first time, and
- data related to the first optotype size and to the measured guessing rate, are stored in said storage memory in correspondence to data of said second time, and
- data related to the second optotype size (OS2.1) and to the remeasured guessing rate, are stored in said storage memory in correspondence to data of said third time, and

said data being furthermore stored in correspondence to an identifier of the user (1).

18. The method according to claim 17, wherein said storage memory stores data of a database (DB) and is accessible through a communication network (NW), by at least one terminal so as to provide at least a history of the evolution of said visual acuity value to a user (4) of said terminal.

19. A digital device (2) comprising a communication interface (COM) comprising at least a screen, a human/machine interface (INT) and a processing circuit configured to implement the method according to any one of the precedent claims.

20. The digital device (2) according to claim 19, wherein output data of the digital device (2) comprises, for a given user (1):

- a visual acuity reference value (VA_ref),
- chosen optotype sizes of displayed optotypes, and
- guessing rates of the displayed optotypes,

the digital device (2) being connected to a communication network (NW) and configured to transmit said output data, through said communication network (NW), to a distant server (SER).

21. A server (SER) connected to a communication network (NW) and comprising a storage memory storing a database (DB) for the implementation of the method according to claim 18, said database (DB) being accessible by at least one terminal and providing at least a history of an evolution of a visual acuity value to a user (4) of said terminal.

22. A computer program product comprising program instruction code stored on a computer-readable medium for the execution of the method according to any one of claims 1 to 18.

FIG. 1

EP 4 209 168 A1

FIG. 2

```
US POS ────── E1
   │
   ▼
DEV POS ────── E2
   │
   ▼
DIST MEAS ────── E3
   │
   ▼
DEV DATA ────── E4
   │
   ▼
DIST CTRL ────── E5
   │
   ▼
NOTIF ────── E6
   │
   ▼
DISPY ────── E7
   │
   ▼
ANSW REC ────── E8
   │
   ▼
EVAL ────── E9
```

# FIG. 3

S0a — VA initialisation VA1.1, OS1.1

S0b — Guessing rate X1.1>THS ?

→ no

↓ yes

S1.1a — VA test at VA1.2=VA1.1 * α, OS1.2

S1.1b — Guessing rate X1.2 < β*THS ?

→ yes

↓ no

...

S1.2a — VA test at VA1.n=VA1.(n-1) * α, OS1.n

S1.2b — Guessing rate X1.n < β*THS ?

→ no

↓ yes

S2 — Research OS1.x, |X1.x − THS| < ε

S3 — END; V_ref = VA1.x, OS_ref = OS1.x

FIG. 4

EP 4 209 168 A1

S4a — VA test VA_ref, OS_ref

S4b — X2.0 == THS ?

yes

S5 — X2.0 < THS ?

no

S51a — VA test VA2.1, OS2.1

S51b — X2.1.1 == THS ?

yes

S61 — X2.1.1 < THS ?

no

yes — VA test VA2.2, OS2.2 — S52a

X2.1.2 == THS ? — S52b

yes

no

S62 — X2.1.2 < THS ?

FIG. 5

FIG. 6

EP 4 209 168 A1

FIG. 7

FIG. 8

Europäisches
Patentamt

European
Patent Office

Office européen
des brevets

# EUROPEAN SEARCH REPORT

Application Number

EP 22 30 5011

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/237347 A1 (ADAPTIVE SENSORY TECH INC [US]) 27 December 2018 (2018-12-27) | 1,19,21, 22 | INV. A61B3/028 |
| Y | * abstract *<br>* claims 1-97; figures 1-28 *<br>* paragraphs [0034] – [0038], [0128] – [0129], [0198] – [0203], [0215] – [0221], [0249] – [0250], [0259] – [0272] * | 2-18,20 | A61B3/032 |
| Y | EP 3 542 704 A1 (TILAK HEALTHCARE [FR]) 25 September 2019 (2019-09-25)<br>* abstract *<br>* claims 1-15; figures 1,2 *<br>* paragraph [0023] – paragraph [0057] * | 2-18,20 | |
| A | US 9 314 154 B2 (UNIV LELAND STANFORD JUNIOR [US]) 19 April 2016 (2016-04-19)<br>* abstract * | 1-22 | |
| A | RU 2 727 873 C1 (FEDERALNOE GOSUDARSTVENNOE AVTONOMNOE UCHREZHDENIE NATSIONALNYJ MEDITS) 24 July 2020 (2020-07-24)<br>* abstract * | 1 | TECHNICAL FIELDS SEARCHED (IPC)<br><br>A61B |
| A | US 9 704 216 B1 (LASKAR SUPRATIM [US] ET AL) 11 July 2017 (2017-07-11)<br>* abstract * | 1 | |

The present search report has been drawn up for all claims

| Place of search | Date of completion of the search | Examiner |
|---|---|---|
| Munich | 24 May 2022 | Tommaseo, Giovanni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 30 5011

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2022

| Patent document cited in search report | | | Publication date | Patent family member(s) | | | Publication date |
|---|---|---|---|---|---|---|---|
| WO 2018237347 | A1 | | 27-12-2018 | AU | 2018289593 | A1 | 13-02-2020 |
| | | | | CA | 3068289 | A1 | 27-12-2018 |
| | | | | CN | 111107779 | A | 05-05-2020 |
| | | | | EP | 3641623 | A1 | 29-04-2020 |
| | | | | JP | 2020525251 | A | 27-08-2020 |
| | | | | US | 2019038125 | A1 | 07-02-2019 |
| | | | | US | 2020113432 | A1 | 16-04-2020 |
| | | | | US | 2021045629 | A1 | 18-02-2021 |
| | | | | WO | 2018237347 | A1 | 27-12-2018 |
| EP 3542704 | A1 | | 25-09-2019 | AU | 2019239531 | A1 | 15-10-2020 |
| | | | | CA | 3094566 | A1 | 26-09-2019 |
| | | | | CN | 112469325 | A | 09-03-2021 |
| | | | | EP | 3542704 | A1 | 25-09-2019 |
| | | | | EP | 3768147 | A1 | 27-01-2021 |
| | | | | JP | 2021519195 | A | 10-08-2021 |
| | | | | US | 2021007599 | A1 | 14-01-2021 |
| | | | | WO | 2019179931 | A1 | 26-09-2019 |
| US 9314154 | B2 | | 19-04-2016 | US | 2014285769 | A1 | 25-09-2014 |
| | | | | US | 2016213243 | A1 | 28-07-2016 |
| | | | | US | 2017143202 | A1 | 25-05-2017 |
| | | | | US | 2021153736 | A1 | 27-05-2021 |
| | | | | WO | 2013059331 | A1 | 25-04-2013 |
| RU 2727873 | C1 | | 24-07-2020 | NONE | | | |
| US 9704216 | B1 | | 11-07-2017 | NONE | | | |

EPO FORM P0459

For more details about this annex : see Official Journal of the European Patent Office, No. 12/82